# DEMANDE DE BREVET EUROPEEN

(11) **EP 0 736 542 A1**
(43) Date de publication de la demande: **09.10.1996**
(21) Numéro de dépôt: 95112019.5
(22) Date de dépôt: 27.09.1991
(51) Int. Cl.: C07K 14/155, C07K 14/16, C07K 14/30

(54) **Peptides inducteurs d'anticorps inhibant des rétrovirus du type HIV et anticorps dirigés contre ces peptides**

(30) Priorité: 27.09.1990 FR 9011951; 26.10.1990 FR 9013324
(62) Demande divisionnaire de: 91919407.6
(71) Demandeur: INSTITUT PASTEUR, F-75724 Paris Cédex 15 (FR)
(72) Inventeur: Montagnier, Luc, F-92350 Le Plessis Robinson (FR); Berneman, Danielle, F-75011 Paris (FR); Guetard, Denise, F-75015 Paris (FR); Bahraoui, Elmostafa, F-78000 Versailles (FR); van Rietschoten, Jurphaas, F-13100 Aix en Provence (FR); Chamaret, Solange, F-75015 Paris (FR); Blanchard, Alain, F-92120 Montrouge (FR)
(74) Mandataire: Gutmann, Ernest

(57) **Abrégé**

L'invention concerne des peptides conténant des séquences d'acides aminés issus de mycoplasmes, notamment de la protéine Mg Pa de l'adhésine de M. genitalium, et plus particulièrement des polypeptides qui ont une homologie de séquence avec des protéines ou glycoprotéines de HIV. Un peptide représentatif est caractérisé par la séquence G V V S T P L V N L I N G Q. Ces peptides - et les anticorps correspondants - sont applicables à la prévention ou à l'inhibition des infections par HIV.

## Description

L'invention concerne des peptides inhibant ou diminuant l'infectiosité de retrovirus du type HIV et des anticorps dirigés contre ces peptides. Dans ce qui suit l'expression "peptides" recouvre tout aussi bien des peptides ou polypeptides, naturels ou synthétiques, des fragments de protéines ou des polypeptides portant ou non des groupements de substitutions caractéristiques de ceux que l'on retrouve chez certaines protéines de structure, par exemple des groupes de glycosylation ou tous peptides pour lesquels une substitution en acides aminés dans la séquence ne modifie pas substantiellement l'activité du peptide telle que décrite dans l'invention.

Font partie de l'invention tous les peptides dont les séquences d'aminoacides (ou en acides aminés) sont issues des protéines de mycoplasmes ainsi que les séquences en aminoacides contenant lesdits peptides et qui sont susceptibles d'induire in vivo ou in vitro (pour les techniques de production in vitro d'anticorps on peut se référer aux travaux de PLUCKTHUN et al décrits dans **Science** 1988, 240 p 1038) la production d'anticorps protecteurs aptes à inhiber ou diminuer in vitro l'infectiosité de HIV, notamment à réduire le développement de l'infection par HIV de lymphocytes T4 par rapport au développement de l'infection observé dans des cultures témoins, en l'absence de ces peptides ou de leurs anticorps spécifiques, voire même à l'inhiber totalement dans les conditions expérimentales décrites plus loin. Cette réduction de l'infection est par exemple appréciée par la réduction correspondante des proportions dosées de transcriptase inverse ou de protéine p24/p25 gag synthétisée dans le cas d'une infection avec HIV-1.

La désignation HIV correspond à l'abréviation de l'expression anglaise "Human immuno-deficiency virus" (Virus d'Immuno-deficience Humaine ou "HIV") telle que définie dans **Nature**, 1988, Vo. 321, p. 10 (COFFIN, J. et al).

Plusieurs auteurs ont montré que les peptides de Mycoplasma pneumoniae étaient reconnus par des anticorps de patients atteints de pneumonies (J. Clin Microbiology, 1990, 28(6), p. 1194-1197, Jacobs E. et al) mais les séquences décrites n'indiquent aucune capacité des anticorps dirigés contre elles à inhiber l'infection à HIV.

Les peptides selon l'invention sont caractérises par des séquences d'acides aminés correspondant à des séquences contenues dans des protéines de mycoplasmes ou dérivées de celles-ci, tout en conservant leur capacité à induire la synthèse d'anticorps inhibant in vitro l'infection, par HIV-1 ou HIV-2, de lymphocytes T ou d'autre types cellulaires permissifs au HIV.

De manière préférée les peptides présentent un pourcentage d'isologie (ou substitution conservative) d'au moins 60% avec des séquences d'acides aminés des protéines issues de HIV.

Des peptides préférés de l'invention contiennent de 5 à 25 acides aminés, notamment de 8 à 15 acides aminés, qui présentent préférentiellement le susdit pourcentage d'isologie avec des séquences d'acides aminés issus d'une protéine de HIV, notamment la protéine gag, les glycoprotéines d'enveloppe, ou encore de la protéine nef d'un HIV (Tableau I).

Compte-tenu de la variabilité génétique des HIV, il apparaît immédiatement que les peptides préférés sont ceux qui présentent un pourcentage d'isologie d'au moins 60%, voire de 70%, avec des régions conservées des glycoprotéines ou protéines de HIV, notamment HIV-1 et HIV-2. Par l'expression "région conservée" de glycoprotéines ou protéines ou peptides de HIV, on entend toutes séquences peptidiques d'au moins 10 acides aminés qui se correspondent mutuellement dans des variants de HIV et présentant un pourcentage d'homologie d'au moins 30% avec les protéines de mycoplasmes, conformément à la définition qui sera donnée de cette expression dans ce qui suit.

Parmi les peptides préférés de l'invention on citera ceux qui contiennent des séquences d'acides amines correspondant à des protéines de structure de mycoplasme et, de manière préférentielle, les séquences qui contiennent des sites actifs d'adhésines de mycoplasme, en particulier de M. pneumoniae, de M. genitalium (souche de référence G37) ou de M. pirum, etc. Font également partie des peptides selon l'invention les peptides correspondant à des protéines de structure de mycoplasme telles que les protéines adhésines et qui présentent les pourcentages d'isologie sus-indiqués avec les séquences gp 160 env, gp 120 env, gp 41 env, p 27 nef et p 55 gag de HIV-1 ou gp 300 env, gp 140 env, p 31 nef, p 55 ou gp 36 env de HIV-2. On désigne ici sous l'expression "adhésine" un composant membranaire des mycoplasmes, responsable de l'adhésion de celui-ci à des supports inertes ou à des cellules.

On parle, dans cette demande de brevet, d'homologie ou d'isologie de séquence entre une séquence d'acides aminés issue d'une protéine de HIV et une séquence issue d'une protéine de mycoplasme lorsque, les représentations symboliques en lignes de séquences d'acides aminés issues de deux protéines ayant été placées l'une au dessous de l'autre, on voit apparaître - au besoin au prix d'un décalage de certaines sous-séquences au sein de l'une de ces séquences les unes vis à vis des autres, de façon à permettre des espacements entre ces sous-séquences qui, respectivement, n'excèdent pas la longueur (représentée par un "tiret" : " - " de l'emplacement correspondant à un acide aminé unique -,
- soit, dans le cas des homologies, une identité entre deux acides aminés placés au droit l'un de l'autre (ou l'un au-dessus de l'autre)
- soit dans le cas des isologies, une appartenance à la même famille, des deux acides aminés au droit l'un de l'autre dans les deux séquences ou sous-séquences respectives, cette famille étant choisie parmi l'une des sept familles suivantes (familles isologiques) :
   (1) R K
   (2) A G W
   (3) L F P M V I
   (4) H
   (5) S T Q N C
   (6) E D
   (7) Y.

Par exemple il y aura isologie lorsqu'au droit d'un acide aminé A dans la représentation symbolique de la première séquence (ou sous-séquence de cette première séquence) se trouve un acide aminé G ou W (famille (2) ci-dessus) dans la représentation symbolique de la seconde séquence.

Deux peptides respectivement issus d'une protéine de mycoplasme et d'une protéine de HIV sont dits présenter une "isologie" ou "homologie" de 30%, lorsque 30% de leurs acides aminés respectifs se trouvent dans la relation sus-définie d'isologie ou d'homologie.

D'autres peptides préférés seront encore indiqués dans la description d'exemples préférés qui suit.

Les peptides préférés sont d'une part ceux qui, issus d'une protéine de structure de mycoplasme ou dérivés de celles-ci, induisent la synthèse d'anticorps inhibant l'infection à HIV et d'autre part ceux dont la séquence comporte une homologie d'au moins 30%, de préférence même une homologie d'au moins 70% avec des séquences correspondantes issues de protéines de HIV. Il peut encore être noté que la longueur préférée de la séquence - au sein de celles qui répondent à la condition sus-indiquée d'induire des anticorps protecteurs contre un HIV - peut également être dictée par le pourcentage d'isologie et, le cas échéant, encore davantage par le pourcentage d'homologie.

Comme cela est rapporté plus loin, l'invention concerne plus particulièrement des séquences peptidiques courtes (comprises entre 5 et 25 acides aminés) de protéines de mycoplasmes qui présentent des pourcentages d'homologie supérieurs à 70% entre elles. Naturellement les peptides constitués par les séquences contenues dans les protéines correspondantes des HIV font également partie de l'invention et, à ce titre, doivent être considérés comme couverts par les revendications.

L'invention découle de deux hypothèses qui ont été faites l'une visant à perturber le contact entre cellules et mycoplasmes susceptibles d'agir en cofacteur du HIV lors d'une infection par ce virus d'une cellule et la seconde hypothèse étant basée sur l'interaction des mycoplasmes avec HIV-1 chez un sujet infecté qui pourrait mettre en jeu des séquences peptidiques de ces deux microorganismes présentant un certain degré d'isologie, sinon d'homologie. En effet, les infections à mycoplasmes sont très fréquentes. Plusieurs articles du groupe de LO ont indiqué la présence de mycoplasmes en localisation intracellulaire dans des tissus isolés de patients infectés par HIV-1 (1) (2) (3), en l'absence de toute correlation dans ces articles entre l'infection à HIV et la présence de mycoplasma fermentans à localisation intracellulaire. L'effet cytopathogène du virus peut être réduit in vitro par des antibiotiques actifs sur les mycoplasmes, d'où l'hypothèse d'une interaction voire d'une coopération entre HIV et une population de mycoplasmes lors de l'infection in vitro de cultures de lymphocytes T et d'autres cellules permissives au HIV (4), bien que l'utilisation de tels antibiotiques ne conduise pas à une éradication totale des mycoplasmes infectant des lymphocytes T.

La présente invention a conduit à la détermination de la structure de peptides issus de protéines de mycoplasmes susceptibles d'induire la synthèse d'anticorps inhibant une infection à HIV dans une culture de lymphocytes T ou d'autres cellules permissives à HIV. L'une des catégories de peptides selon l'invention est définie comme regroupant les peptides présentant outre leur capacité à induire la synthèse d'anticorps inhibant ou diminuant une infection à HIV, la capacité d'avoir une homologie ou une isologie avec des protéines de HIV. Pour cela une recherche d'homologie a été faite entre les séquences des sites actifs des adhésines de deux mycoplasmes (M. pneumoniae et M. genitalium) et les séquences d'acides aminés codées par les gènes env, nef et gag d'HIV-1 et HIV-2.

L'invention couvre également un procédé de préparation de "molécules hybrides" ou "peptides hybrides" caractérisé; en ce qu'il met en oeuvre la construction de molécules d'acides nucléiques constituées pour partie de séquences nucléotidiques codant pour au moins un peptide appartenant à HIV et, pour partie une séquence codant pour au moins un peptide de mycoplasme selon l'invention. L'invention couvre également les compositions de peptides hybrides liés entre eux de manière covalente ou constituées par un mélange non covalent de peptides de HIV et de peptides de mycoplasmes selon l'invention, non liés entre eux directement de manière covalente.

**TABLEAU I**

| Gène | Protéines codées (Kd) | Fonctions et caractéristiques |
|---|---|---|
| LTR | | Séquences terminales répétées (long terminal repeats) nécessaires à l'intégration du génome viral dans le génome cellulaire et lors de la transcription inverse. |
| TAR | | Séquence nucléaire cible lors de la transactivation par l'ARNm de tat |
| GAG | Protéines internes (« core antigens ou group specific antigen ») | Précurseur myristilé de 55 Kd qui est clivé en 3 protéines |
| | P 18 \| | |
| | P 25 \| p 55 | |
| | P 13 \| | |
| POL | polymérase | Polyprotéine de 110 Dd dont le clivage conduit à 3 protéines : |
| | | - protéase P10 |
| | | - transcriptase inverse P64 |
| | | - endonucléase/intégrase P34 |
| VIF (ou SOR ou gene A ou ORF Q) | P 23 | Facteur viral d'infectiosité |
| VPR (ou ORF R) | P 15 | Région codante située entre VIF et TAT |
| TAT | P 15 | Protéine régulatrice (transactivation de toutes les protéines de virus) |
| REV (ou TRS ou ART) | P 20 | Protéine régulatrice de l'expression des protéines virales |
| VPU | P 16 | Protéine ayant un rôle dans la maturation des particules virales |
| VPU | P 16 | Protéine ayant un rôle dans la maturation des particules virales |
| ENV | GP 120 | Protéine de l'enveloppe virale externe |
| | GP 41 | Protéine transmembranaire |
| NEF (ou 3'ORF ou négative factor) | P 27 | Protéine myristilée de régulation négative de l'expression de protéines virales |
| Note : VPU n'existe pas dans VIH2 et SIV. En revanche, il existe un gène VPX qui code pour une protéine P16 qui aurait un rôle au niveau de la réplication de virus. | | |

L'étude a porté plus particulièrement sur les protéines de structure et en particulier les protéines adhésines de Mycoplasma genitalium et de Mycoplasma pneumoniae.

La séquence nucléotidique du gène de la protéine P1 d'attachement à l'épithélium cilié respiratoire, de M.pneumoniae a été décrite par Inamine et al (6) et un site essentiel pour l'adhésion par Dallo, S.F. et al. (5).

La séquence du gène codant pour l'adhésine de M. genitalium a été décrite par Dallo et al (12) et il a été montré par Morrison-Plummer et al (7) que l'adhésine de M.genitalium partageait des déterminants immunologiques communs avec M.pneumoniae pour la fixation sur la cellule. Cette adhésine est dénommée "MgPa" pour M.genitalium.

Le tableau II présente l'homologie des séquences des adhésines au niveau des régions impliquées dans la cytoadhérence.

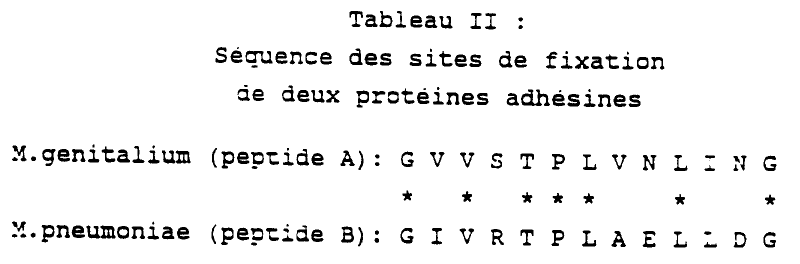

Les astériques * symbolisent l'homologie existant entre les deux sequences alignées.

L'outil informatique mis en oeuvre était un ordinateur DATA GENERAL MV/8000.

Les séquences des adhésines de M. pneumoniae et de M. genitalium non accessibles directement sur banques de données ont été entrees manuellement.

Pour avoir accès aux séquences de protéines rétrovirales on peut également se référer à la source connue sous la désignation "Compilation and Analysis of nucleic acid and aminoacid sequences of human retroviruses and AIDS" (Compilation et analyse de séquences d'acides nucléiques et d'aminoacides de rétrovirus humains et SIDA), 1988-1989, éditée par Gerald Myers et al, Los Alamos, USA, "National Laboratory" (Laboratoire National).

### Critères de tri des données et alignement des séquences

L'algorithme de Lipman et Pearson a été utilisé (9, 10)
a) Pour les recherches d'homologies strictes on a pris comme critère de tri:
   - la présence de deux acides aminés consécutifs homologues ("K-tuple size" 2 ou taille minimum des séquences homologues à partir desquelles l'ordinateur commence sa recherche de sequences plus longues ayant un pourcentage d'homologie minimum choisi par l'opérateur),
   - une "lecture" de 20 en 20 acides aminés (taille de la fenêtre ou "window size"),
   - une pénalité d'espacement maximum entre sous-séquences ("gap penalty") de un.
b) Pour les alignements par isologie, le "K-tuple size" est de 3.

**TABLEAU III**

| Représentation symbolique des acides aminés | | |
|---|---|---|
| Alanine | Ala | A |
| Arginine | Arg | R |
| Asparagine | Asn | N |
| Aspartate | Asp | D |
| Asn + Asp | Asx | B |
| Cysteine | Cys | C |
| Glutamine | Gln | Q |
| Glutamate | Glu | E |
| Gln + Glu | Glx | Z |
| Glycine | Gly | G |
| Histidine | His | H |
| Isoleucine | Ile | I |
| Leucine | Leu | L |
| Lysine | Lys | K |
| Méthionine | Met | M |
| Phenylalanine | Phe | F |
| Proline | Pro | P |
| Sérine | Ser | S |
| Thréonine | Thr | T |
| Tryptophane | Trp | W |
| Tyrosine | Tyr | Y |
| Valine | Val | V |

### Recherches d'homologies entre les adhésines de M. pneumoniae et de M. genitalium et les protéines d'envelope de HIV-1 (BRU) et P55 gag HIV-1 (BRU) et P27 nef HIV-1 (BRU) (résultats)

A titre de référence HIV1 (BRU) a été décrit comme étant HIV1 (LAI) Science, 1991, WAIN-HOBSON et al, 252, p. 961-965, nous avons commencé par effectuer un alignement direct entre la séquence de l'adhésine de M. pneumoniae (1624 acides aminés) et celle de M. genitalium (1444 acides aminés). Ce résultat a montré 723 acides aminés communs, soit 45% d'homologie. Dans l'approche par isologies entre M. pneumoniae et M. genitalium, le pourcentage est de 59% (957 acides aminés/1624).)

### A) Homologies gp 160 env (HIV-1 BRU) et adhésine P1 de M. pneumoniae

1) L'alignement de la séquence de P1 sur la séquence de la protéine de l'enveloppe montre 178 homologies ("matches" ou "coïncidences"). Ceci n'est pas significatif en raison de la large dispersion de ces homologies sur la séquence de ces 2 protéines. D'un point de vue "isologie", les résultats sont assez différents car le nombre de coïncidences est de 328 mais surtout certaines séquences présentent des coïncidences groupés et la dispersion est moins grande. En particulier plusieurs groupes de 8 à 10 coïncidences sont observés tels que : 
M. pneumoniae :                    S G Q N T S T
                                    *   * * * * *
Glycoprotéine d'enveloppe HIV-1 : N A T N T N S S
ou :
M. pneumoniae :              T T T P V F G T S S G N
                             * * *   * * * *   * * *
Glycoprotéine d'en-          C T T A V P W N A S W S
veloppe de HIV-1 :
2) Alignement du site actif de fixation de P1 M.pneumoniae avec la séquence correspondante de la protéine d'enveloppe de HIV-1 ; aucun résultat significatif n'a été obtenu en homologie, du moins en ce qui concerne les séquences en acides aminés testées. En revanche, avec un alignement par substitution conservative ou isologique, on obtient :

env HIV-1 :                KVQKEY-AFFYKLDIIPIDND
                         *** *  ***  *    *** *
p1 (M.pneumoniae) :      KMNDDVDGIVRTPLAELLDGE
                         **   * *** ***   **  *
Mg Pa (M.genitalium) :   KMDGKLTGVVSTPLVNLINGQ
                         **      ***  * * * *
env HIV-1 :              KVQKEY-AFFYKLDIIPIDND

Les alignements montrent 9/21 acides aminés isologues entre HIV-1 et M. genitalium, 13/21 entre les 2 séquences des adhésines des mycoplasmes mentionnés ci-dessus.

Autre isologie retenue :

env HIV-1          :     WFNITNWLWYIKIFIMIVGGLV
                          ** ***         ***  *
site Pg Pa M.genitalium :  GV-VST---------PLVNLIN

Le site actif de fixation présumé de M.genitalium a 9 isologies communes avec une région de la partie transmembranaire de la protéine d'enveloppe du HIV1 (BRU)

### B) Homologies entre p27 nef de HIV-1 (BRU) et P1 de M.pneumoniae

Aucun résultat significatif par la méthode de tri utilisée n'a été obtenu pour les recherches d'homologies strictes et les isologies à partir des chaînes d'aminoacides plus particulièrement étudiées

### C) Homologies entre P55 gag de HIV-1 (BRU) et P1 de M.pneumoniae

Pas de résultat significatif avec la méthode de tri ulilisée.

### D) Homologies entre la gp160 de l'enveloppe de HIV-1 (BRU) et Mg Pa de M.genitalium

On observe 165 coïncidences dispersés sur toute la longueur. On remarque des séquences de 8 acides aminés, dont 6 acides aminés sont identiques, soit une homologie de 75%.

M.genitalium :        L I V T V P I V
                       * * * *     * *
gp 160 env HIV-1 :     L I V T R - I V

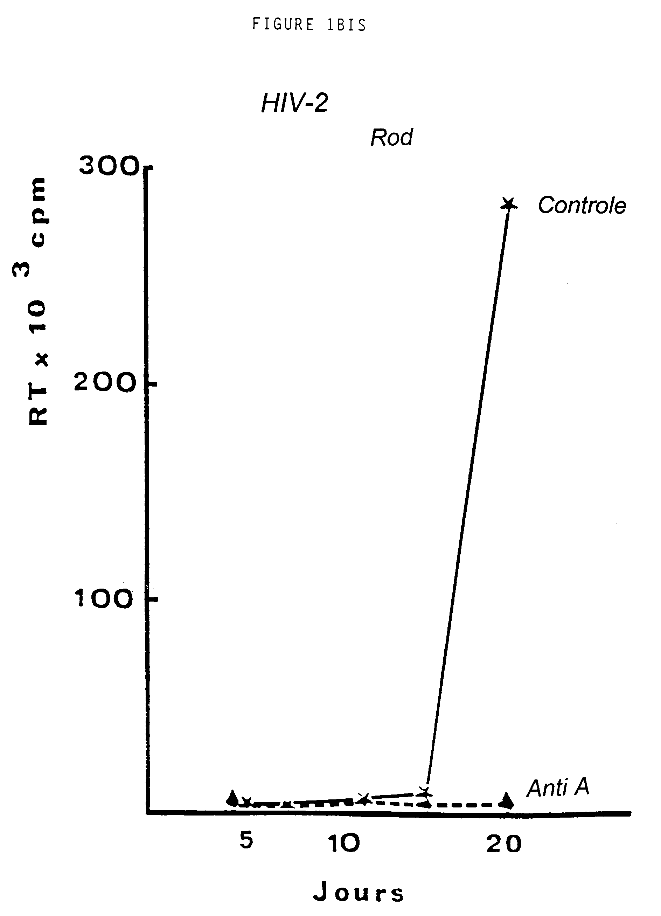

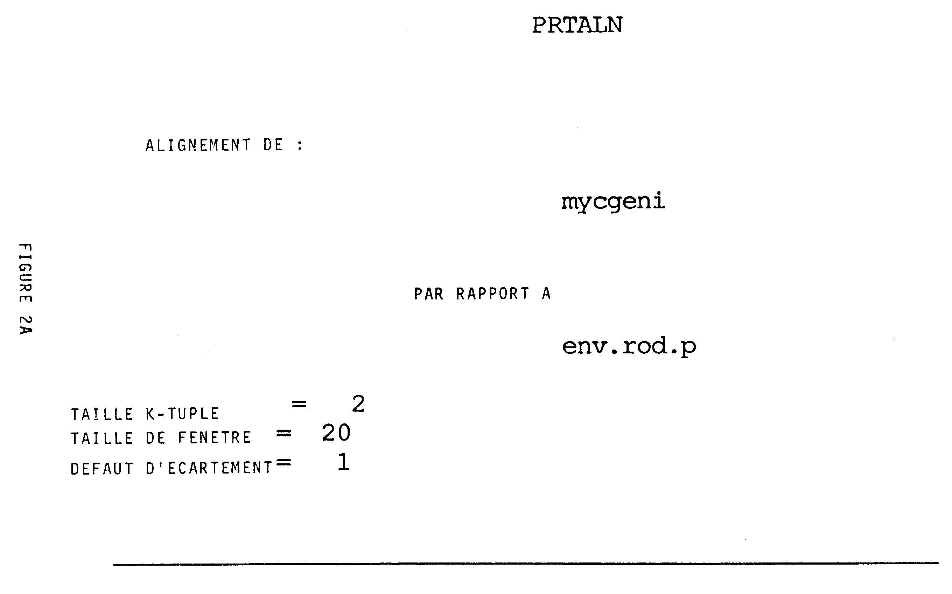

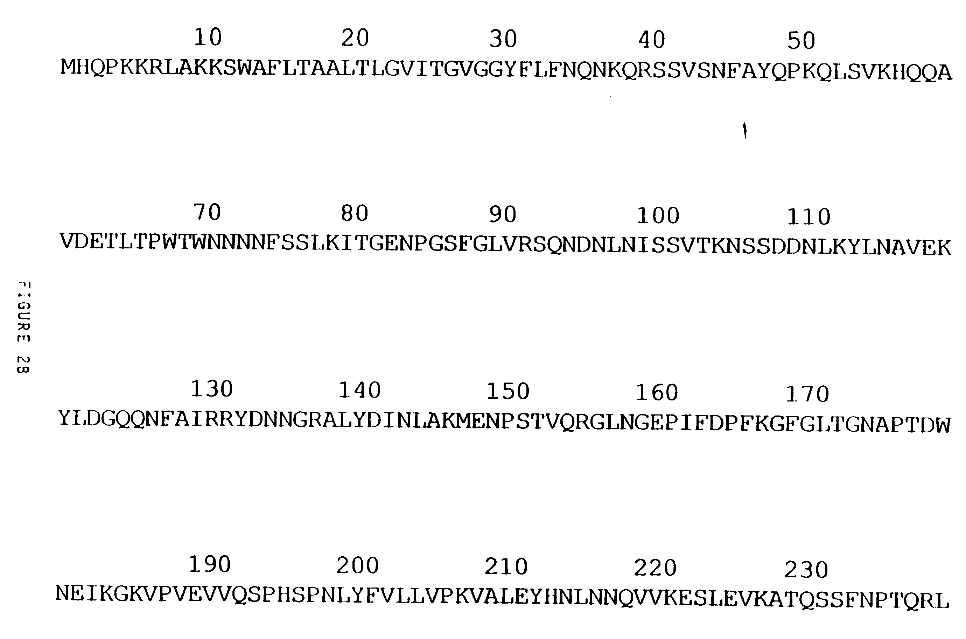

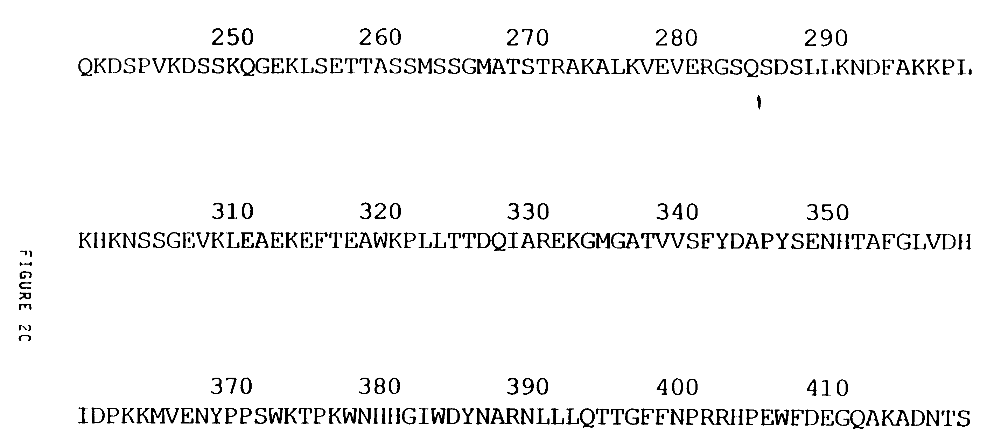

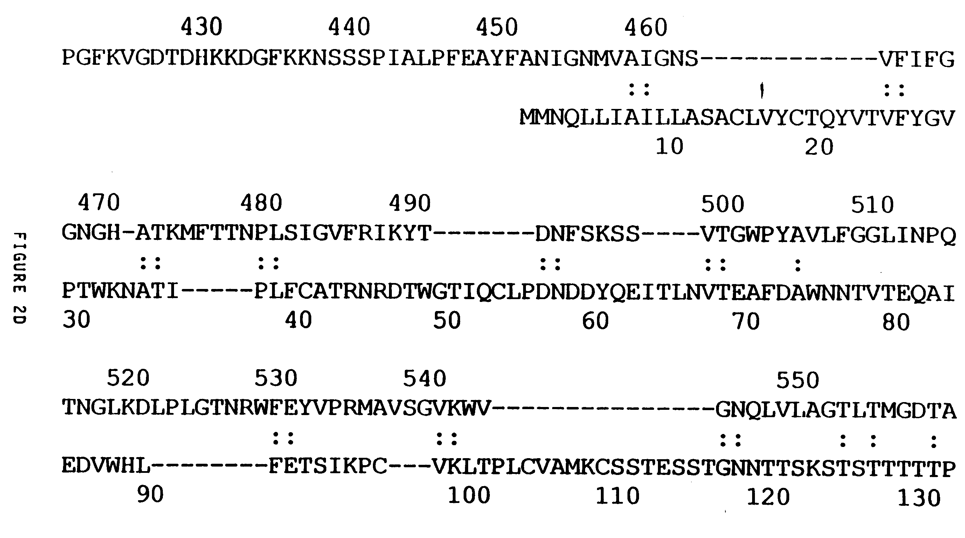

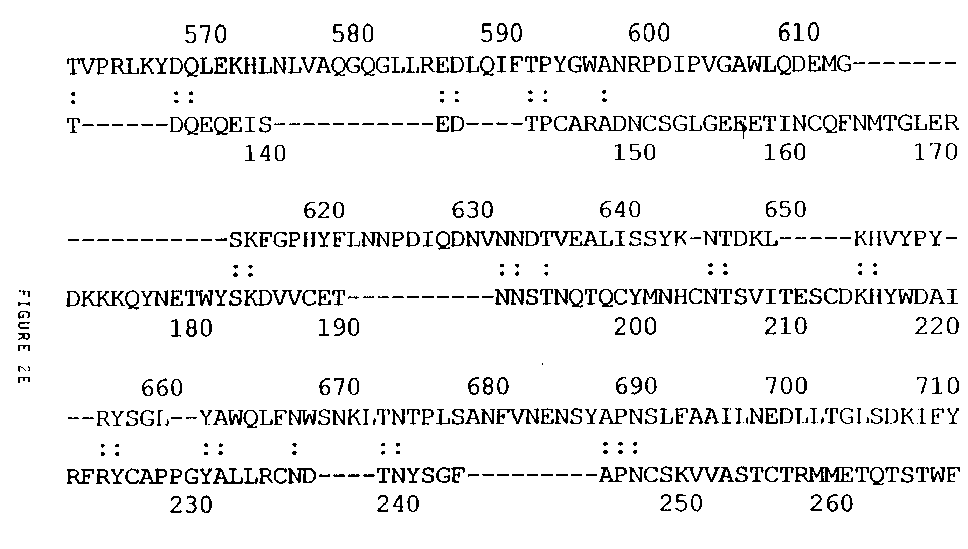

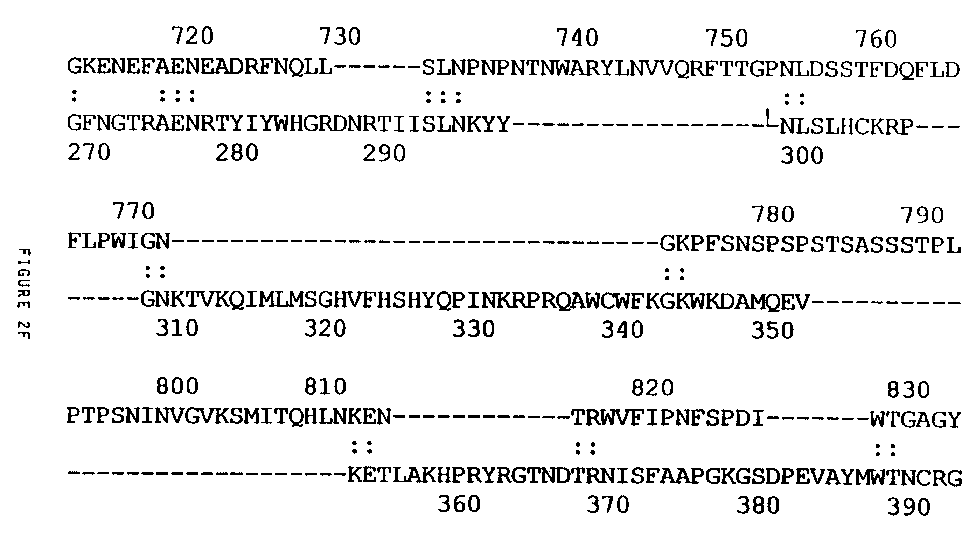

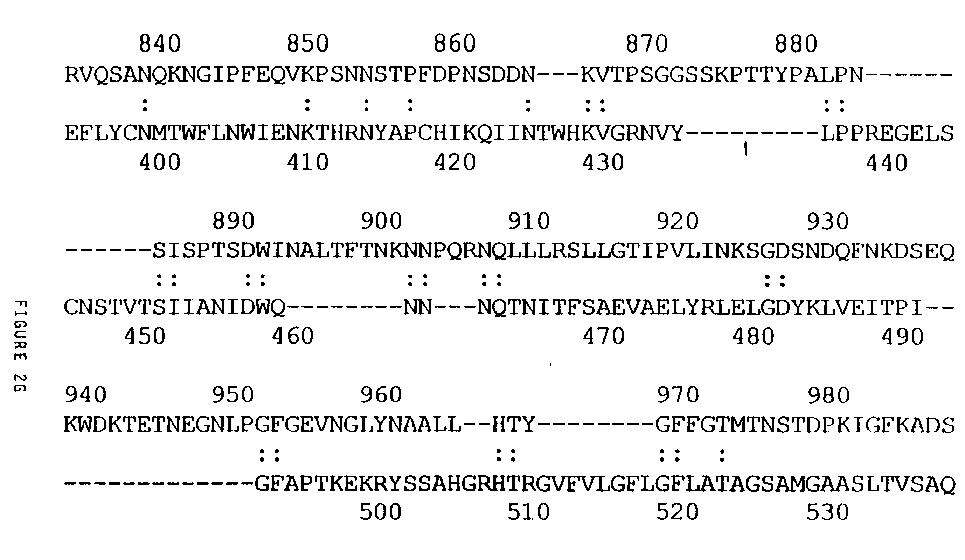

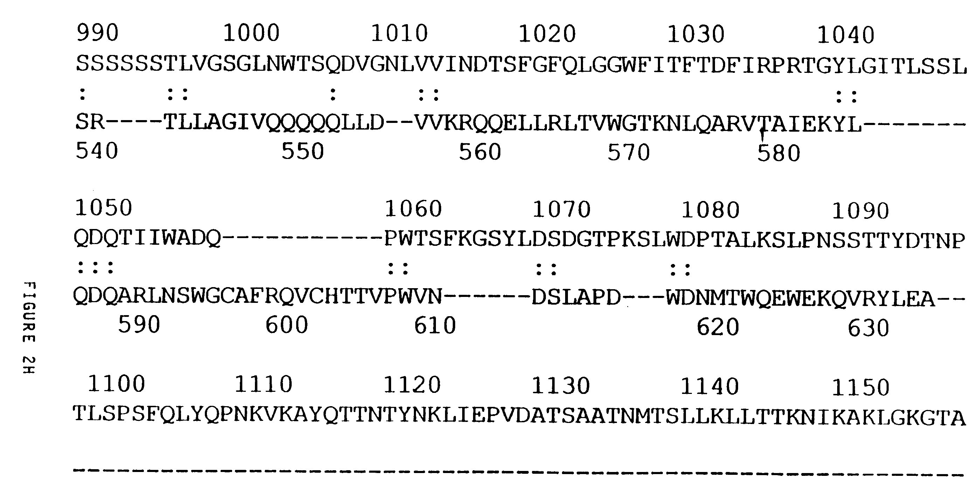

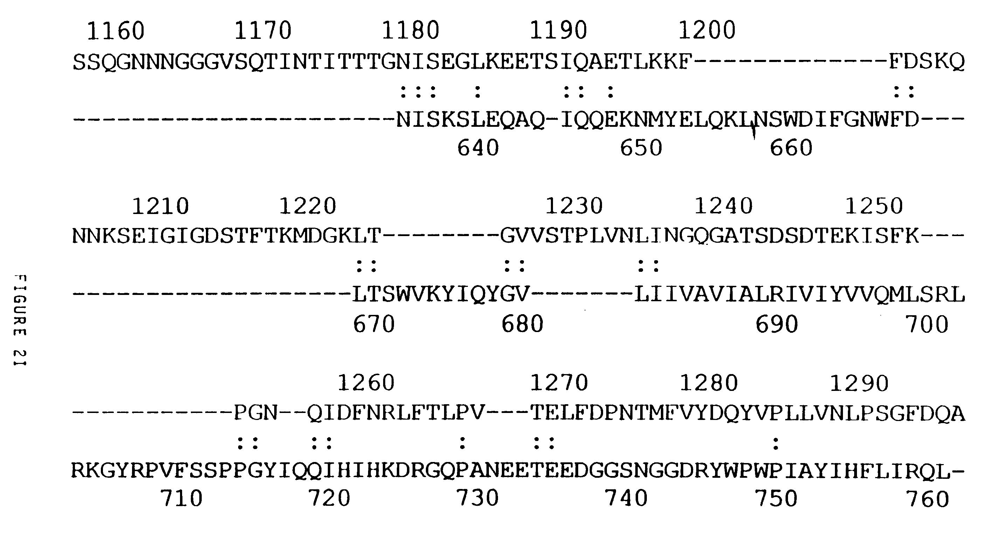

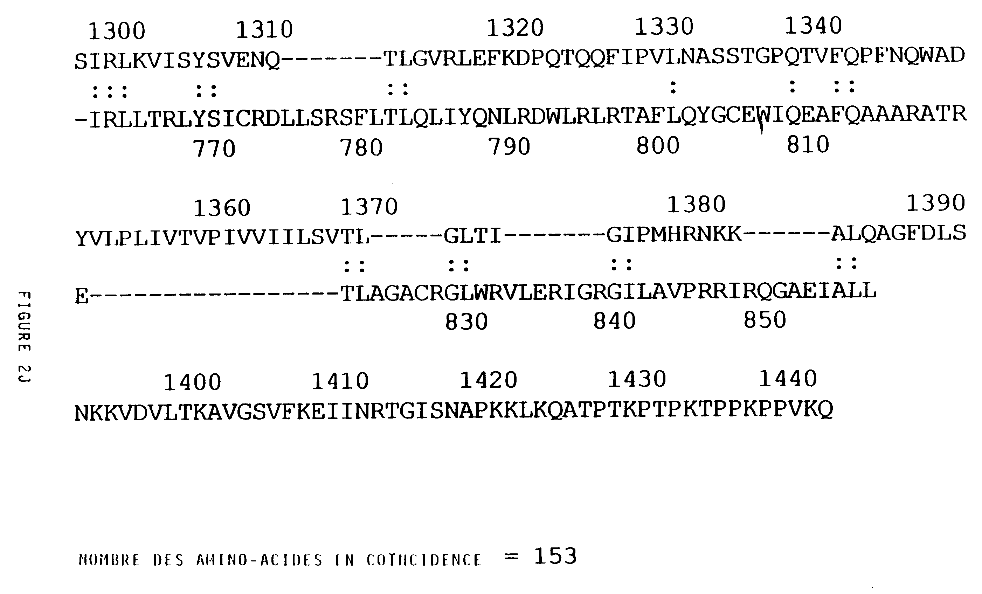

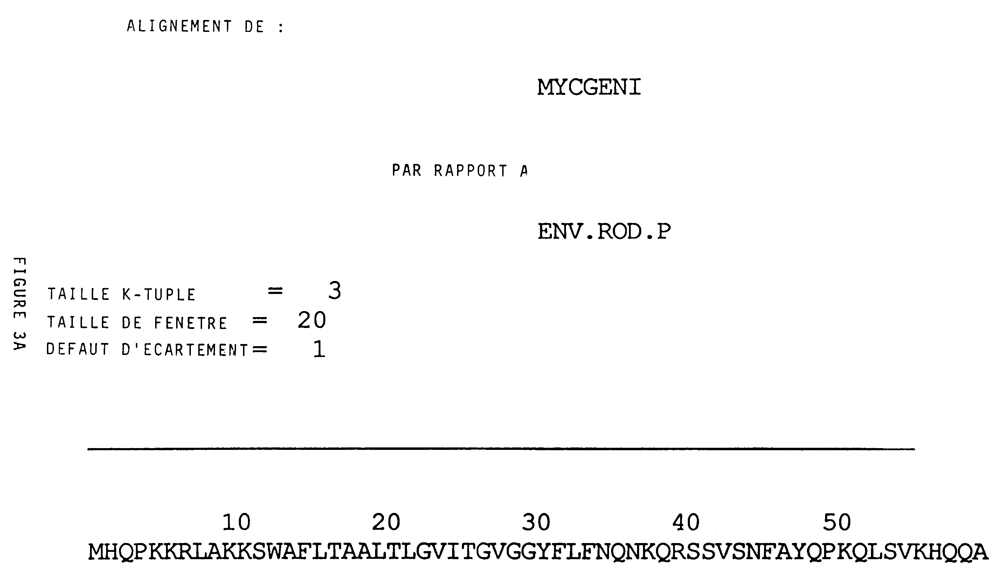

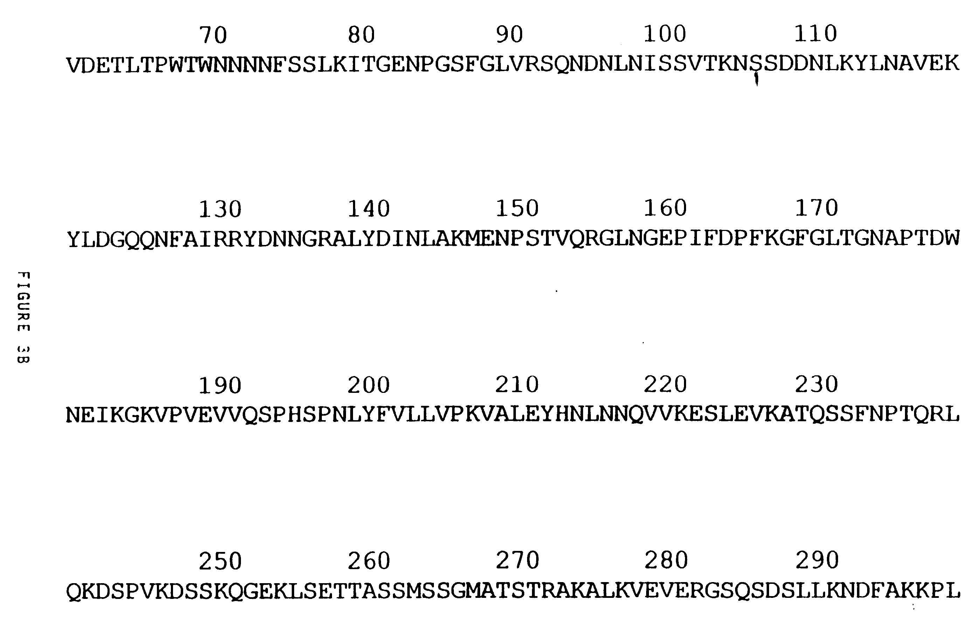

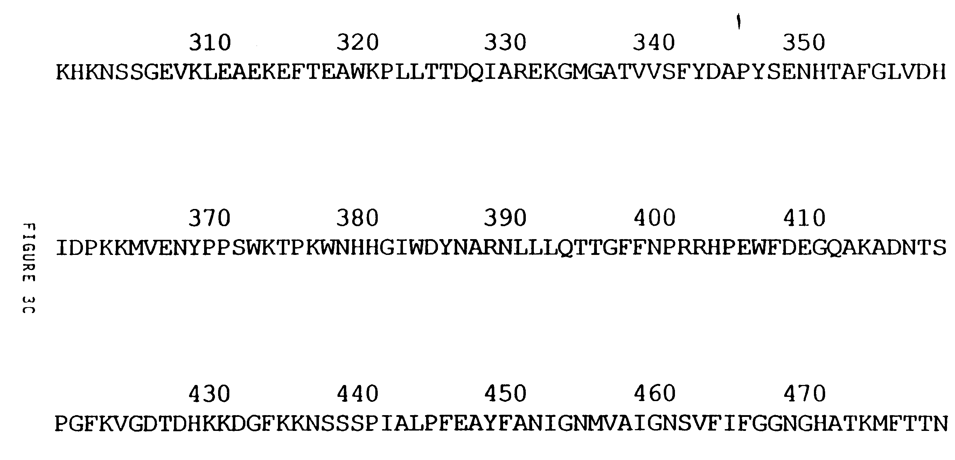

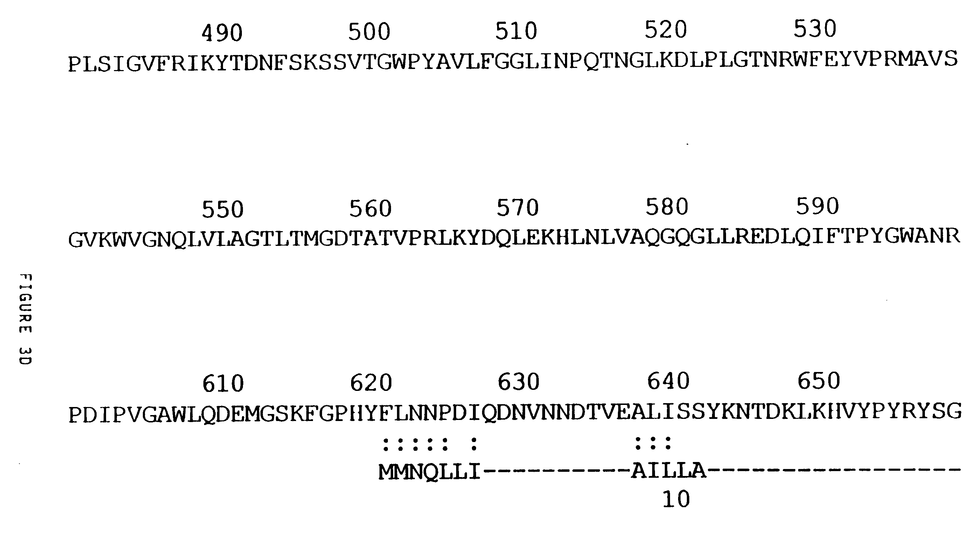

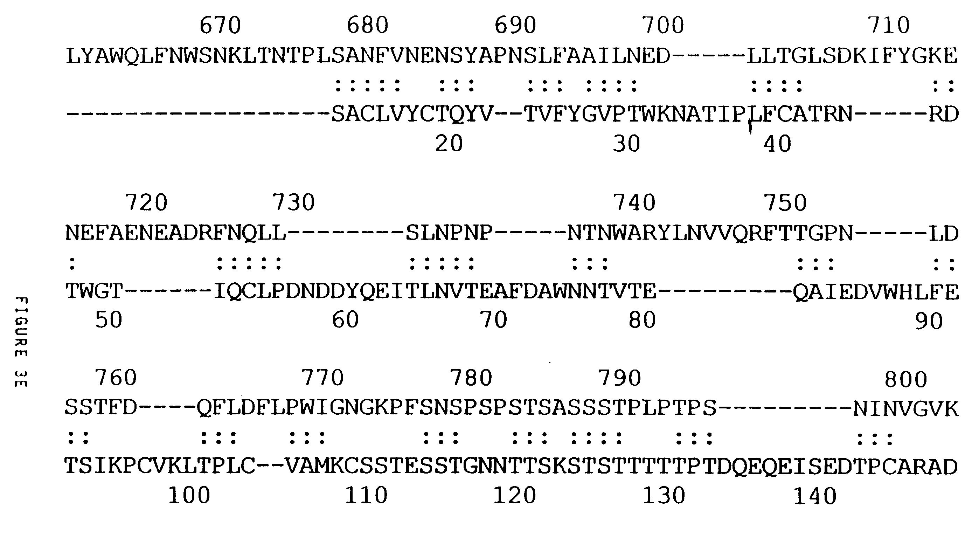

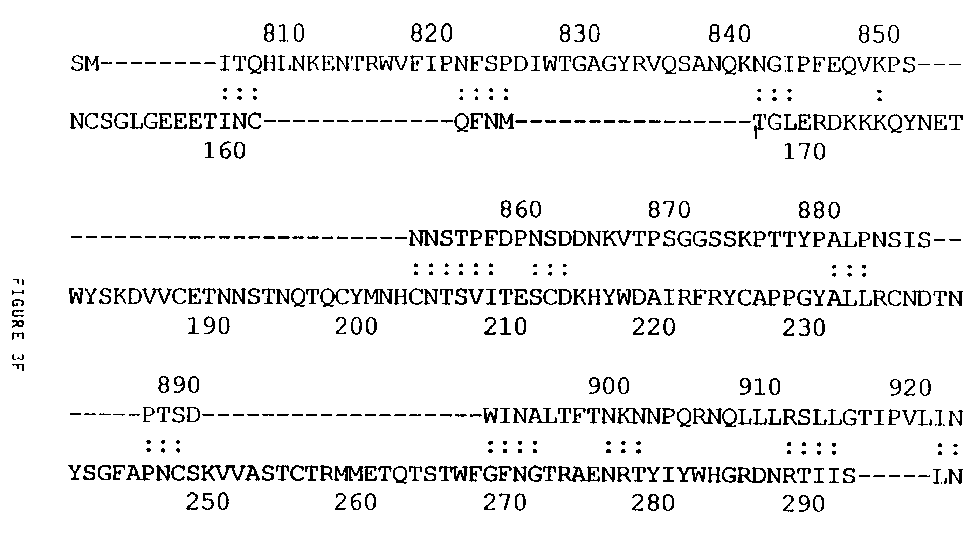

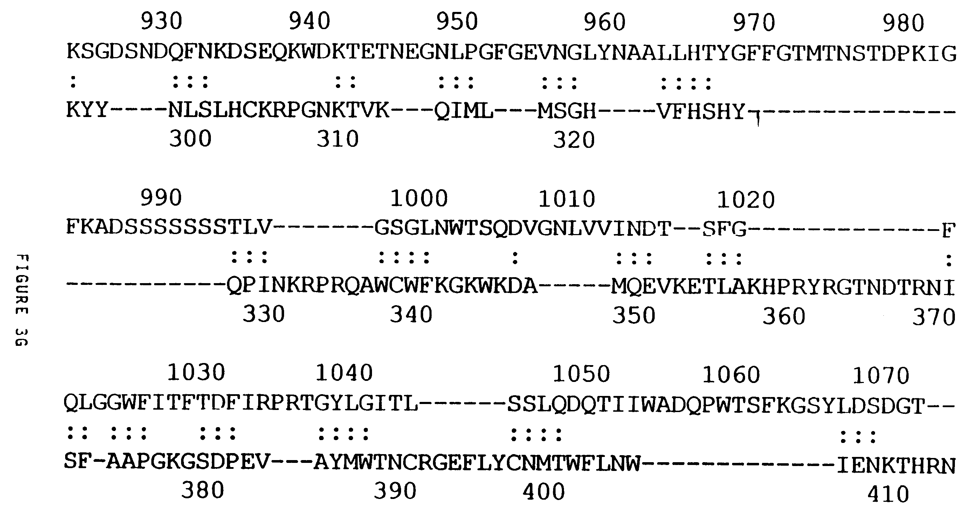

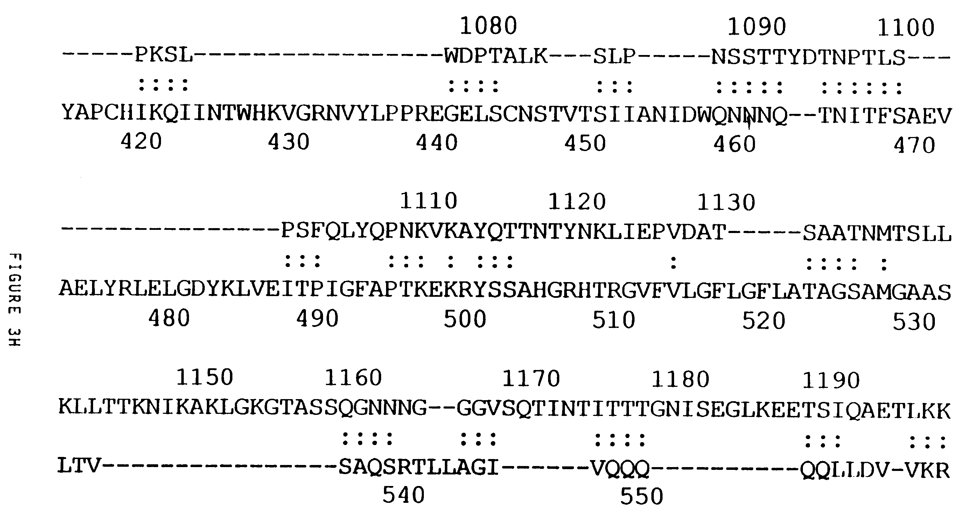

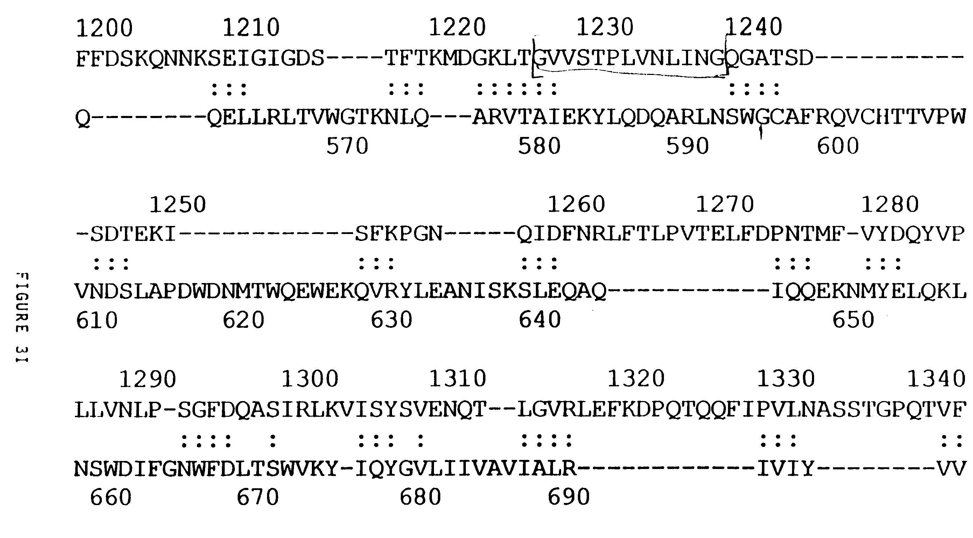

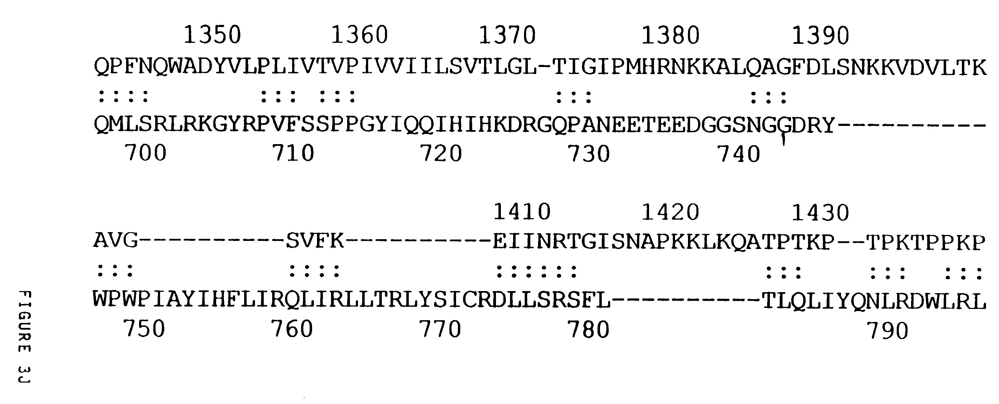

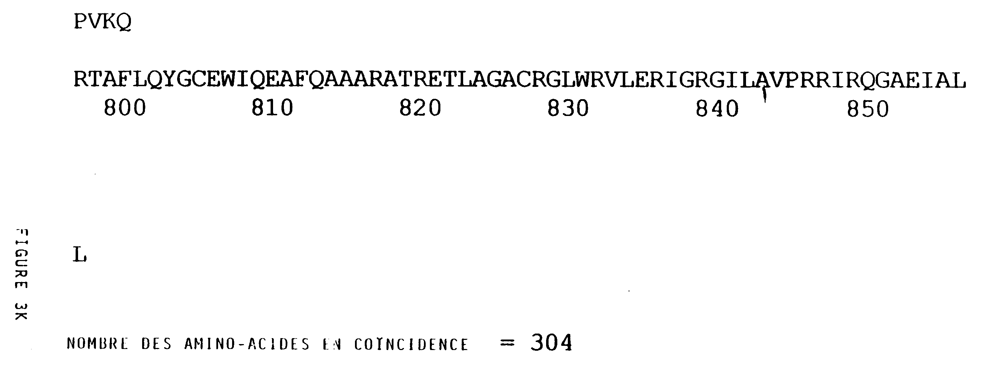

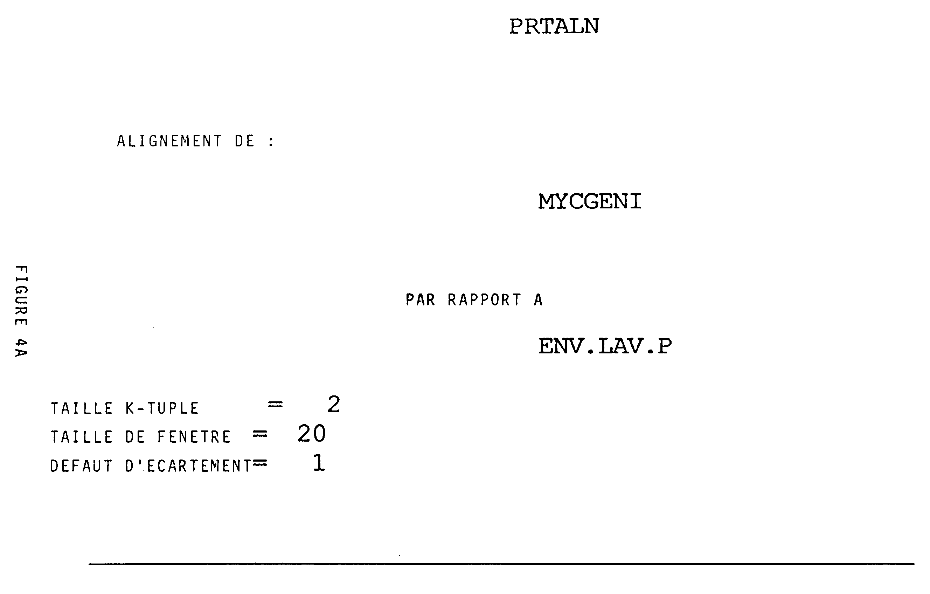

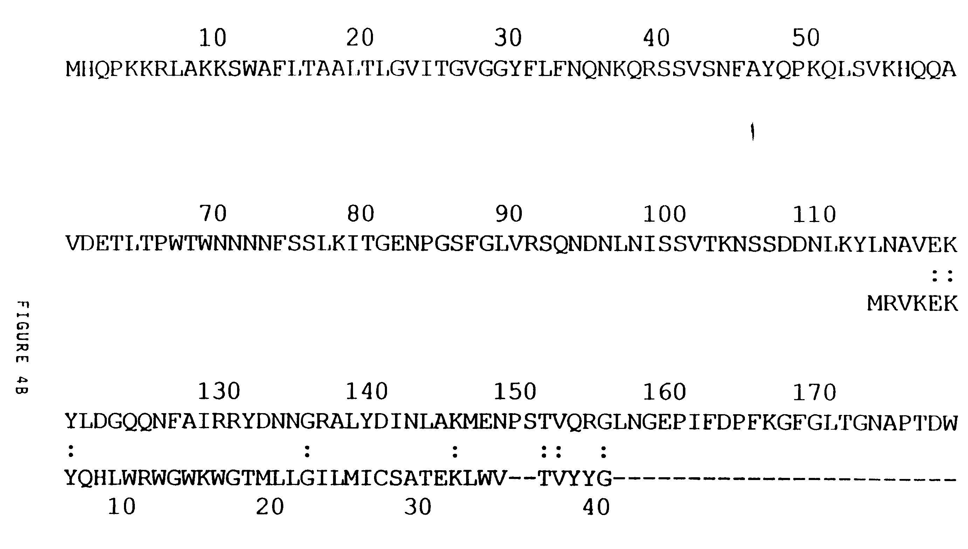

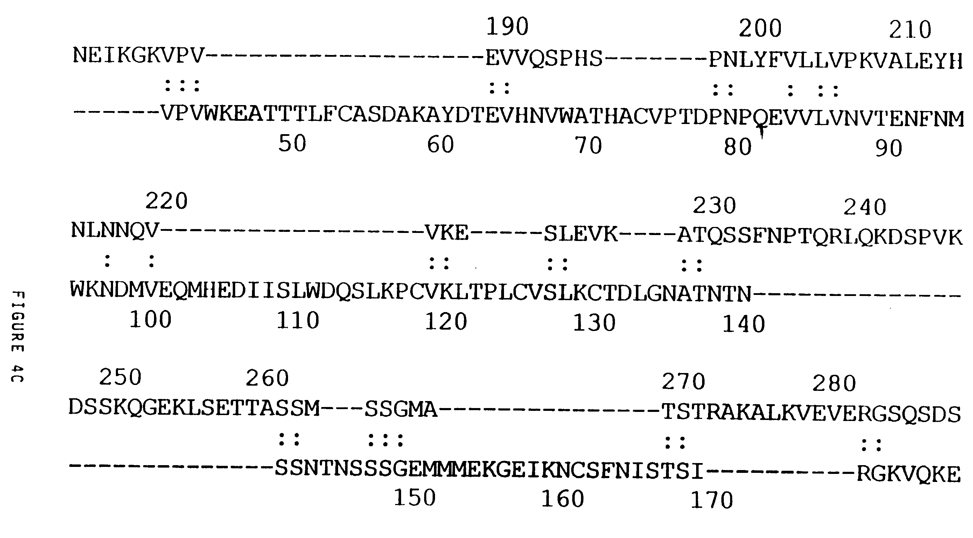

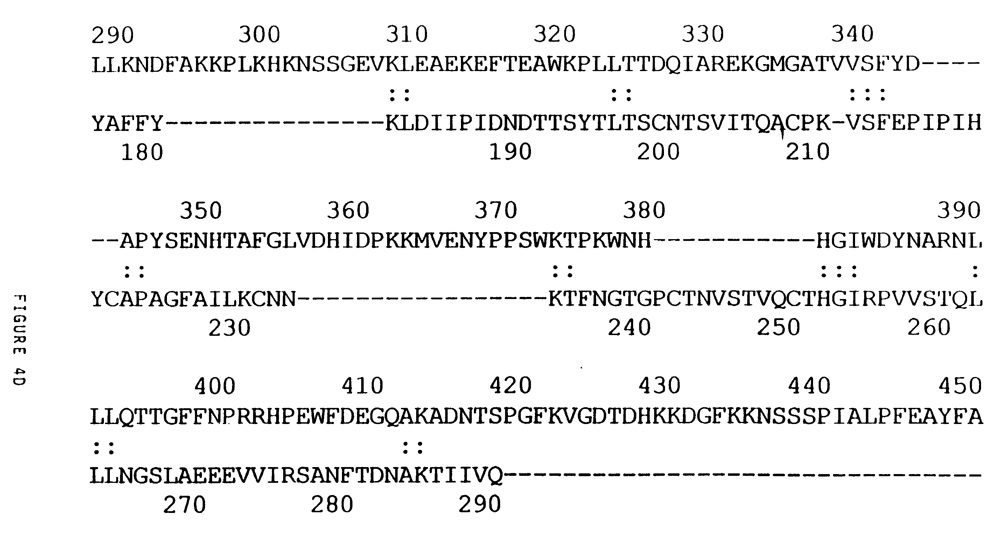

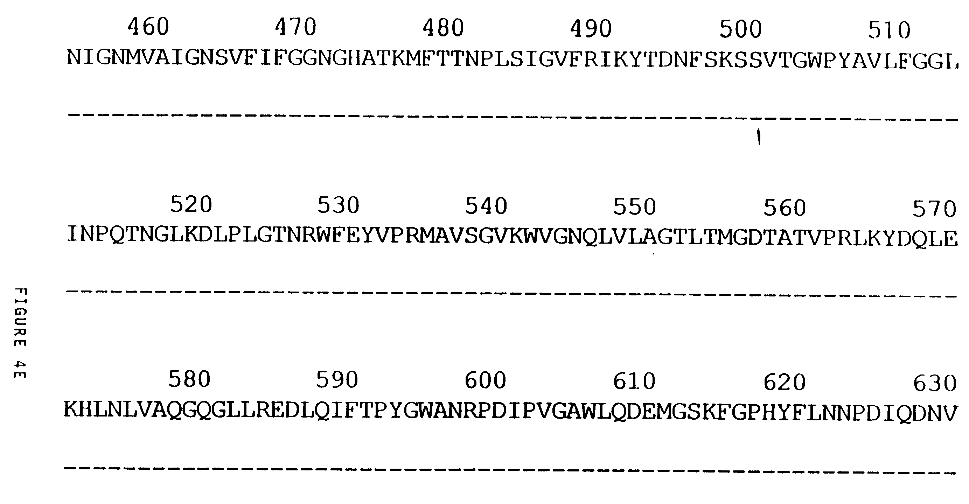

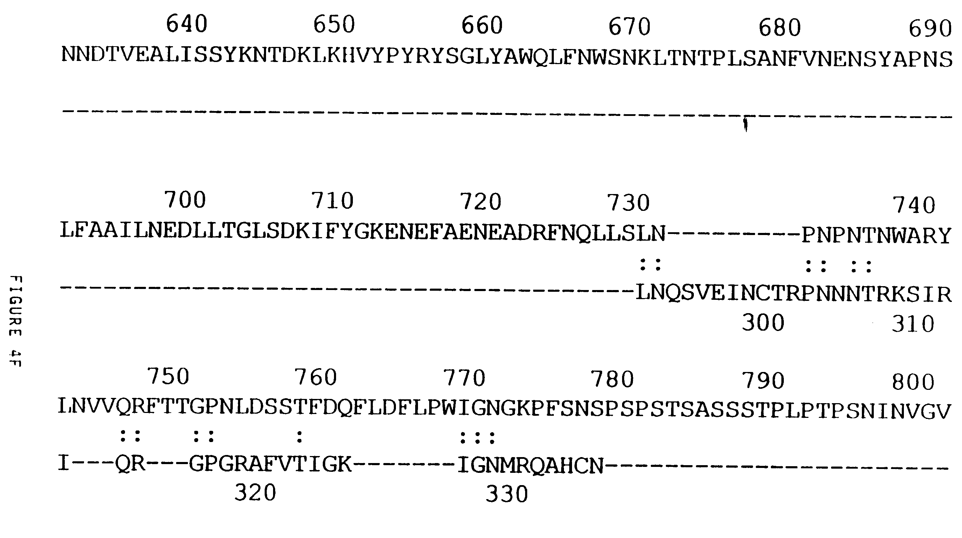

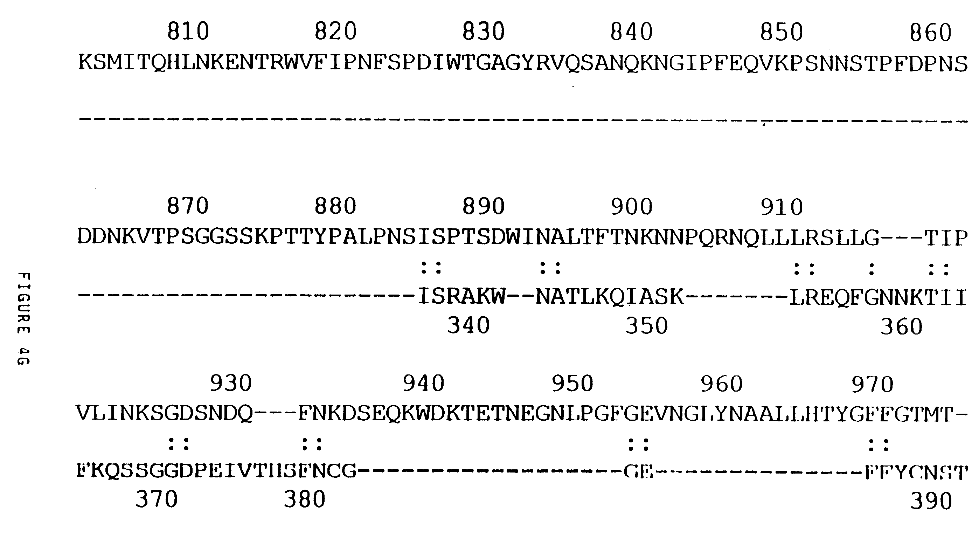

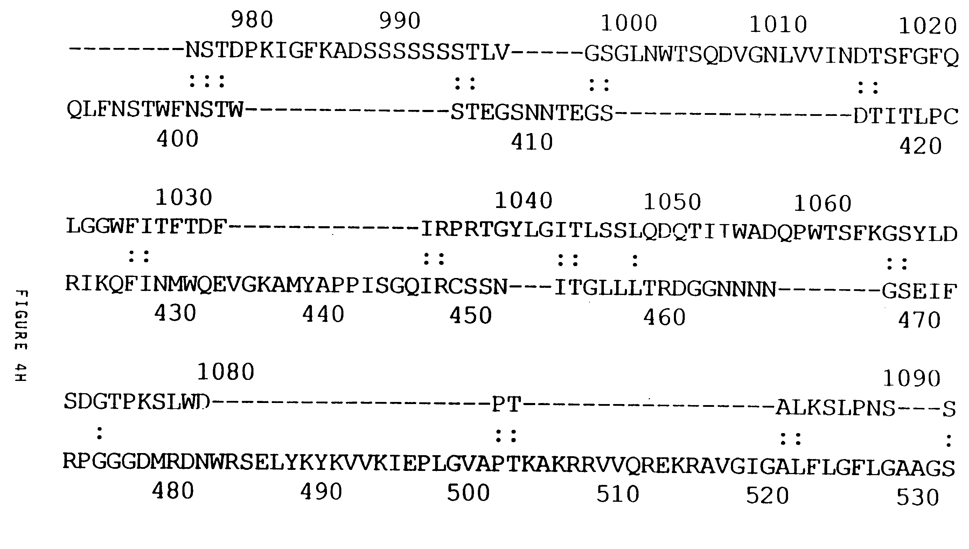

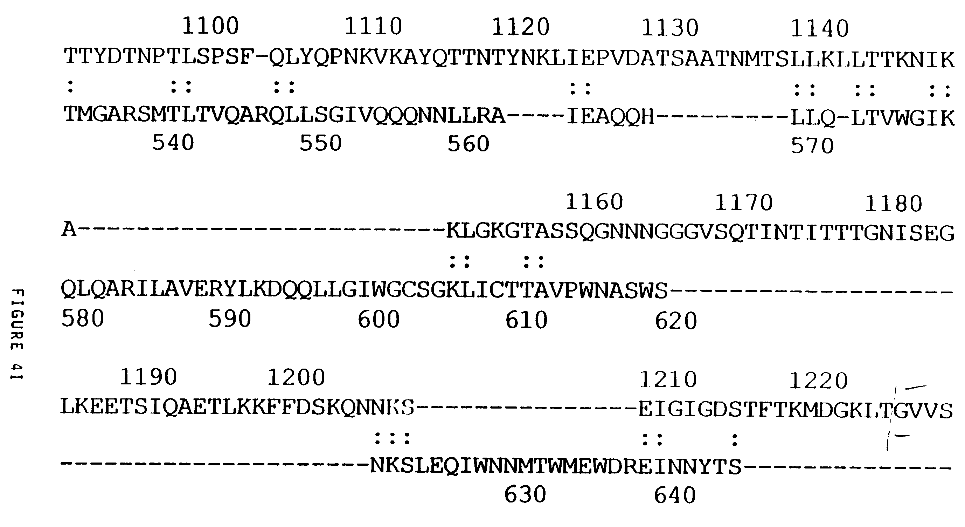

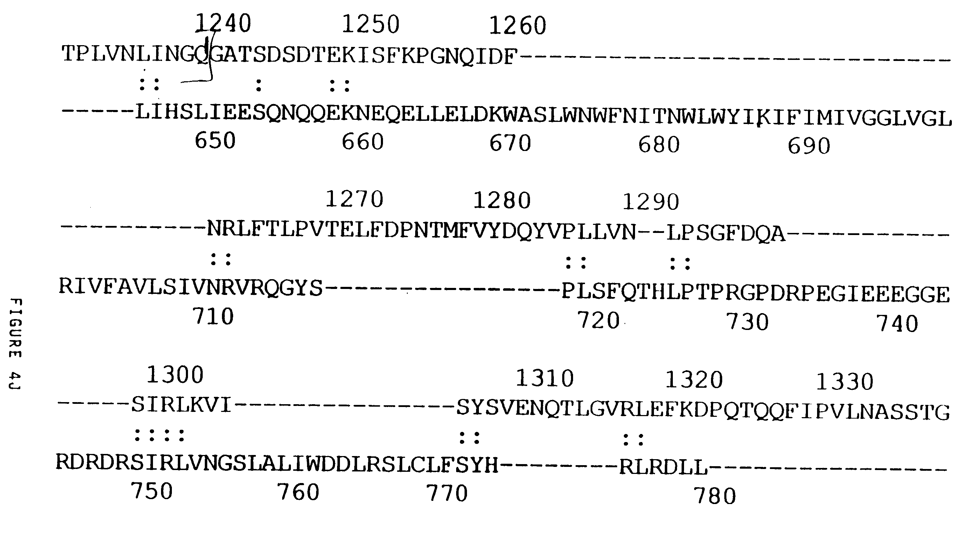

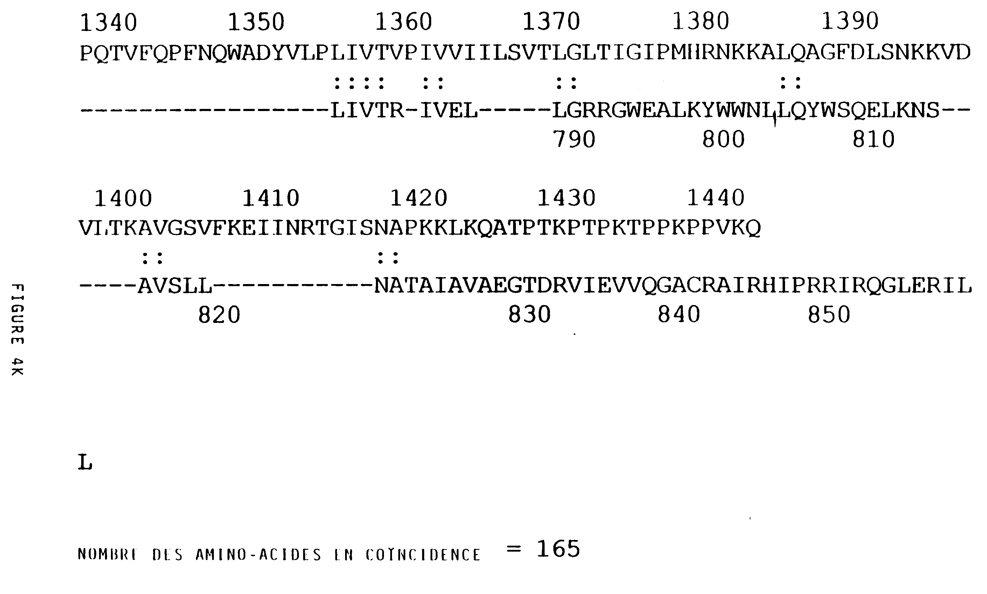

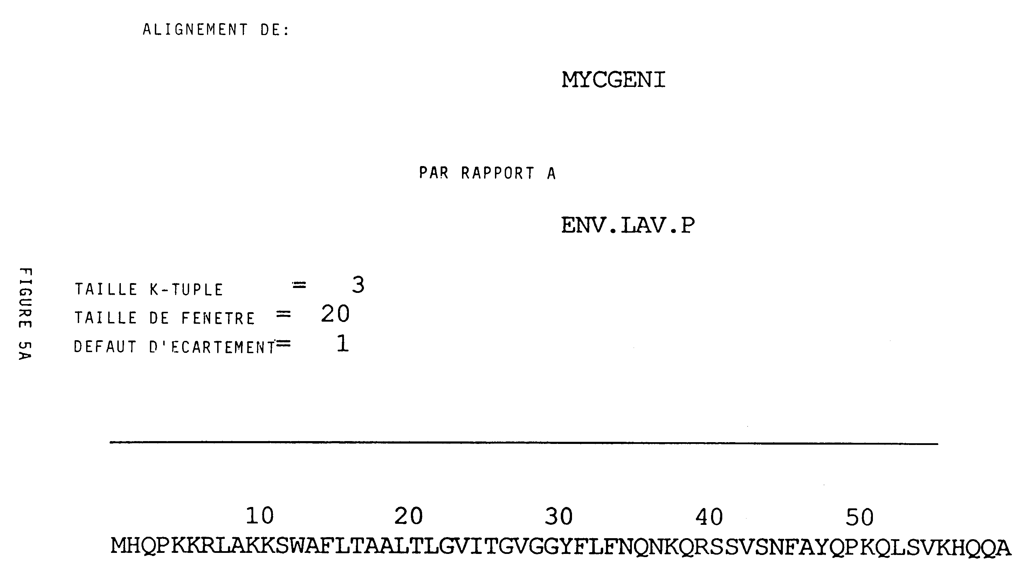

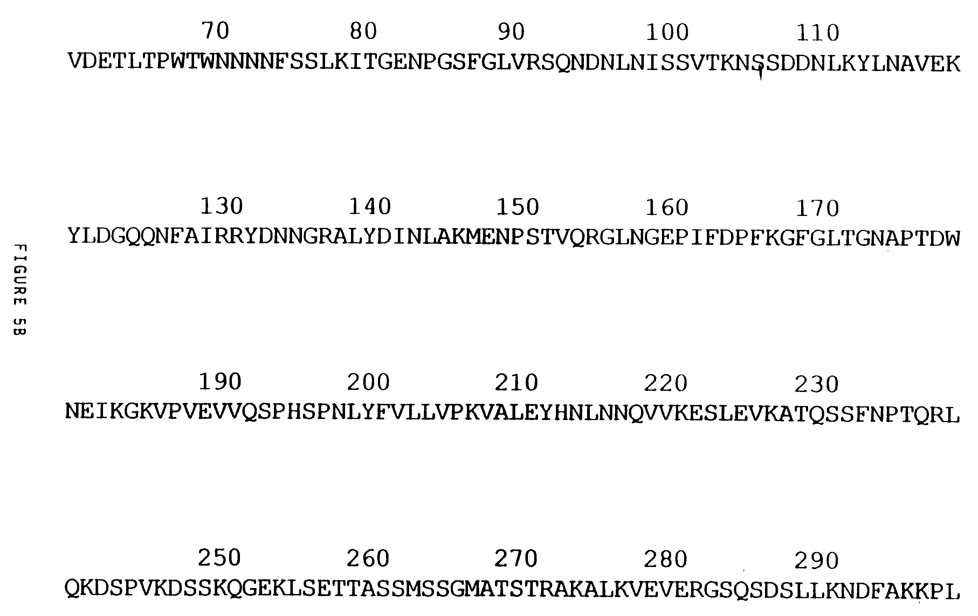

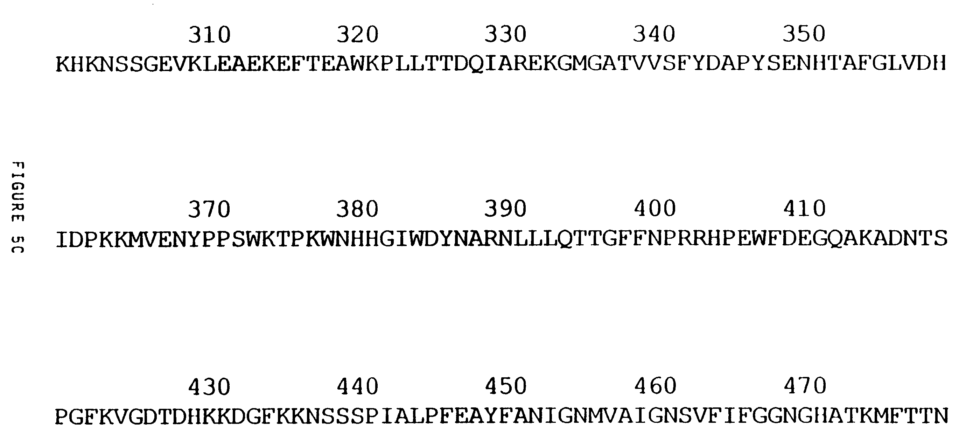

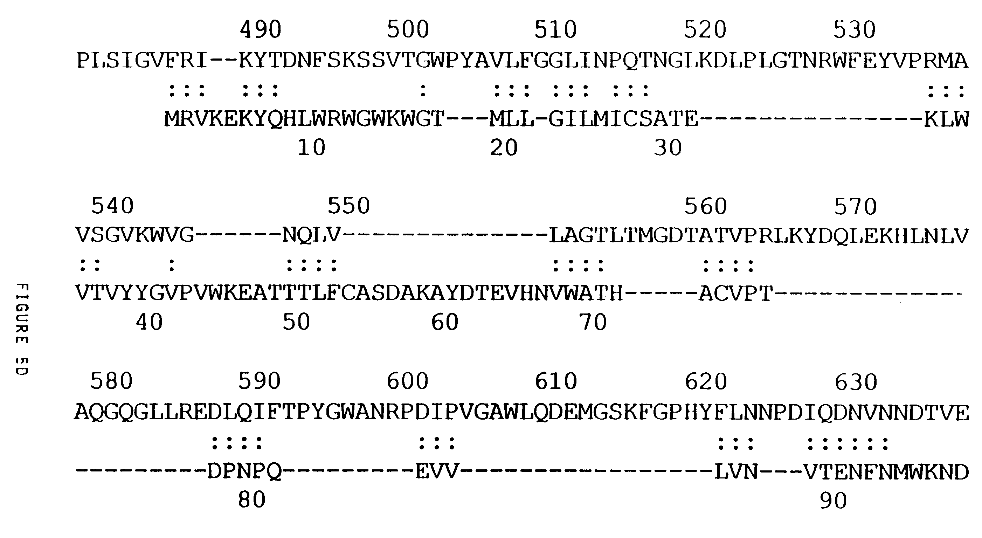

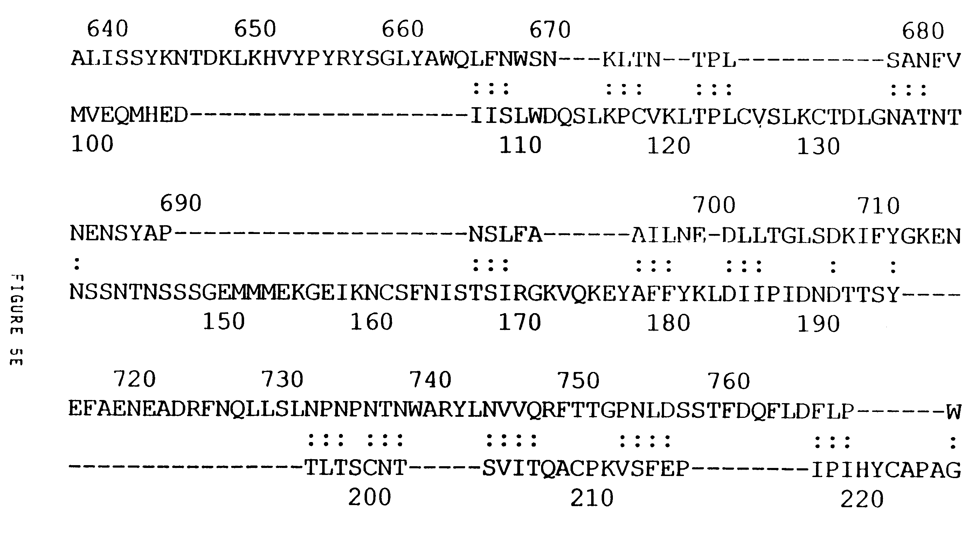

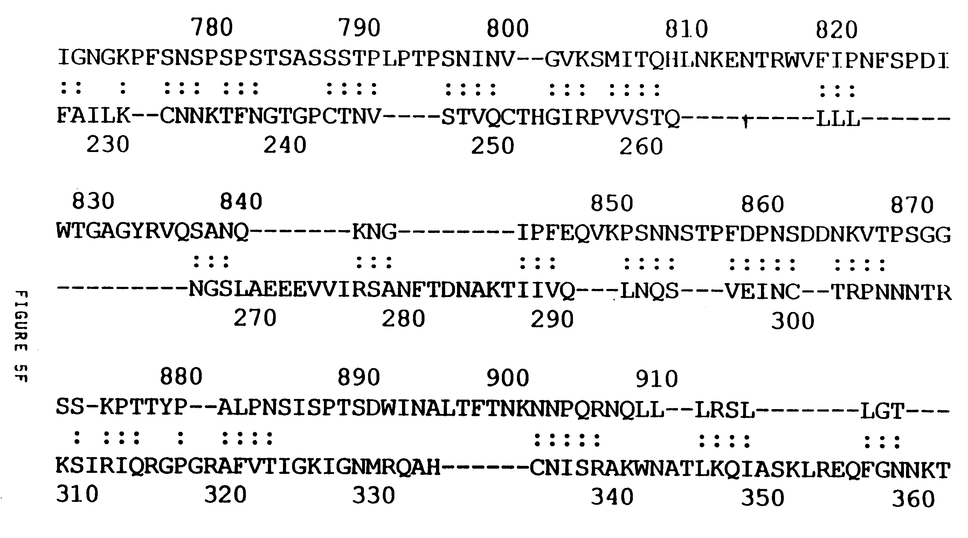

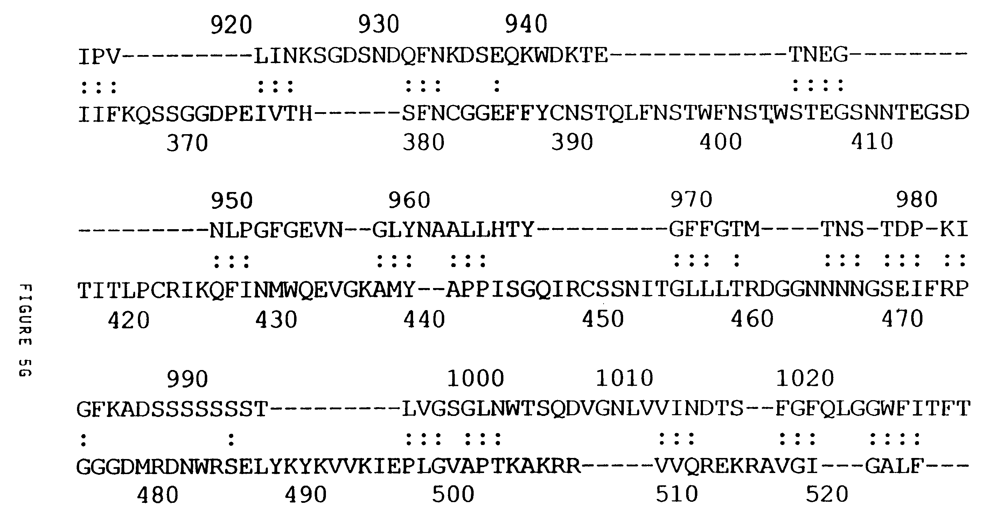

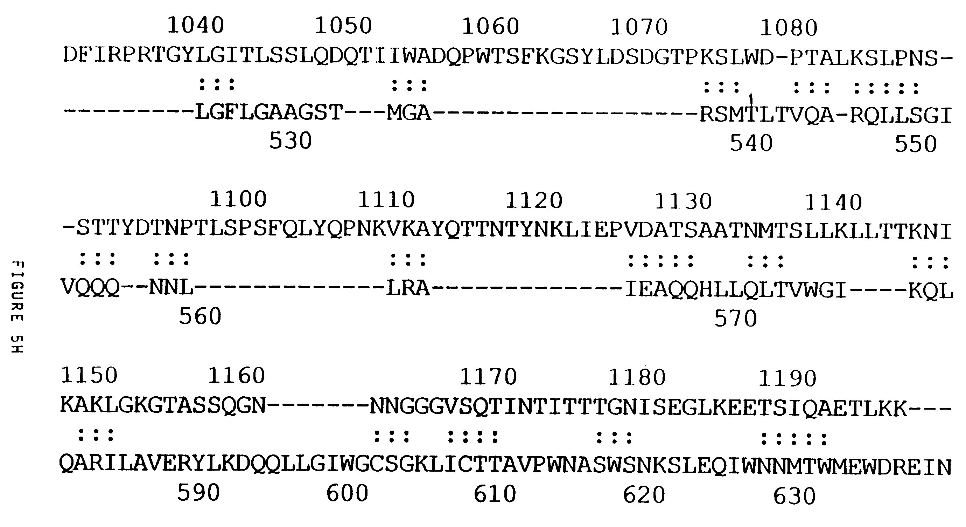

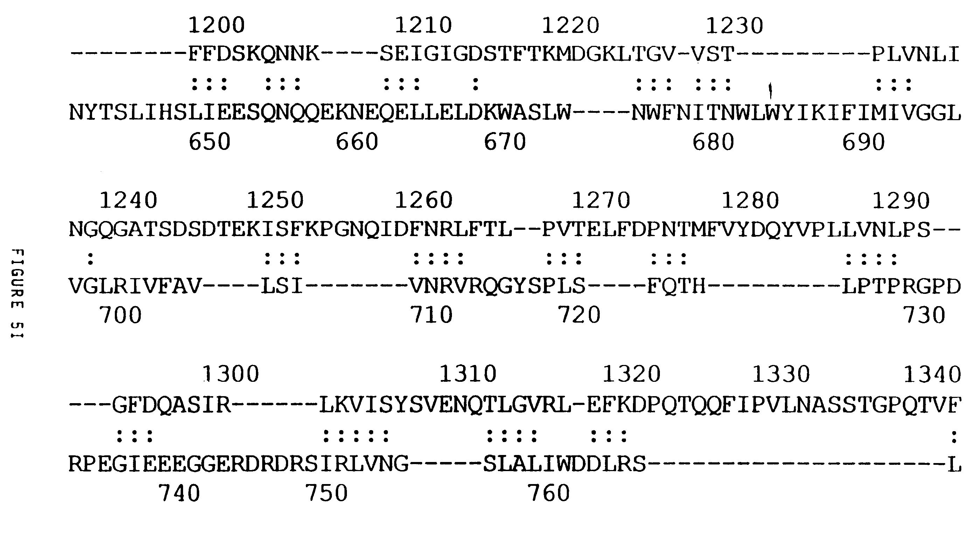

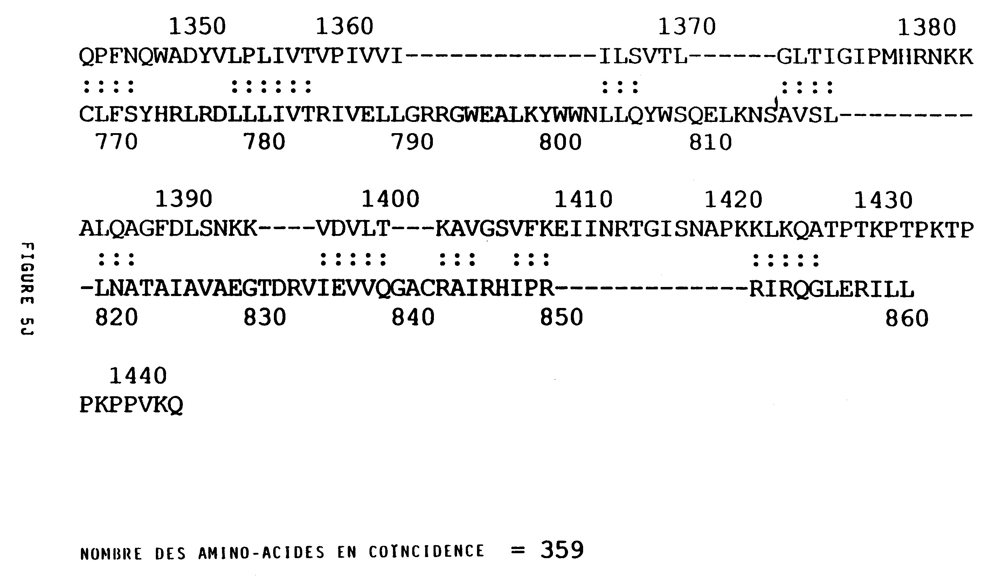

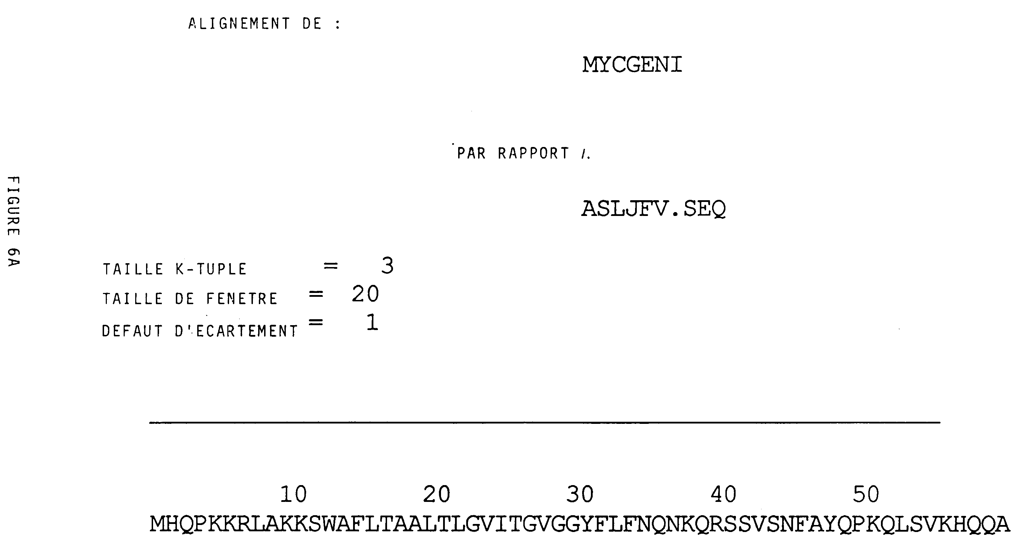

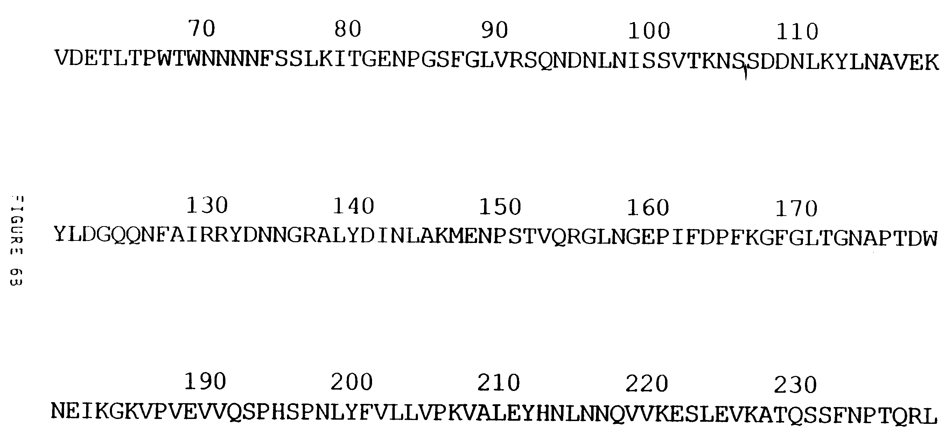

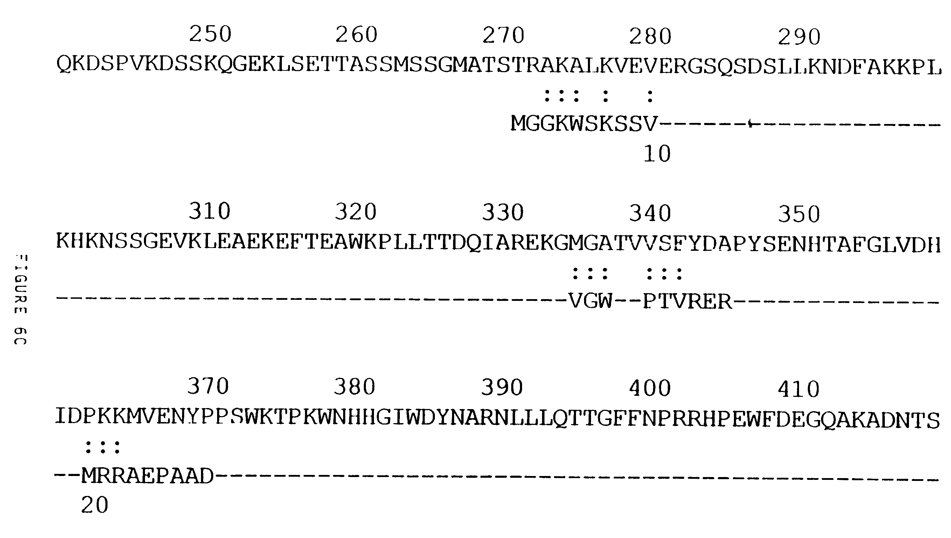

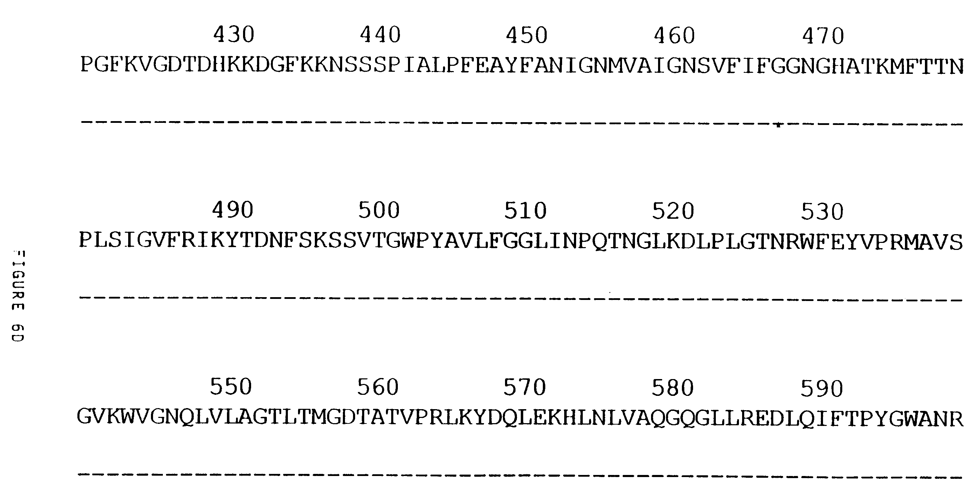

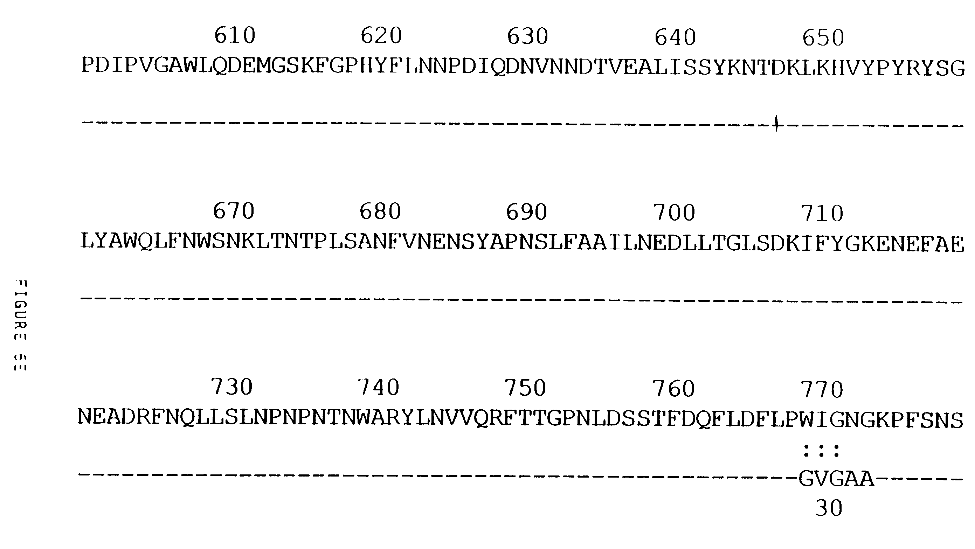

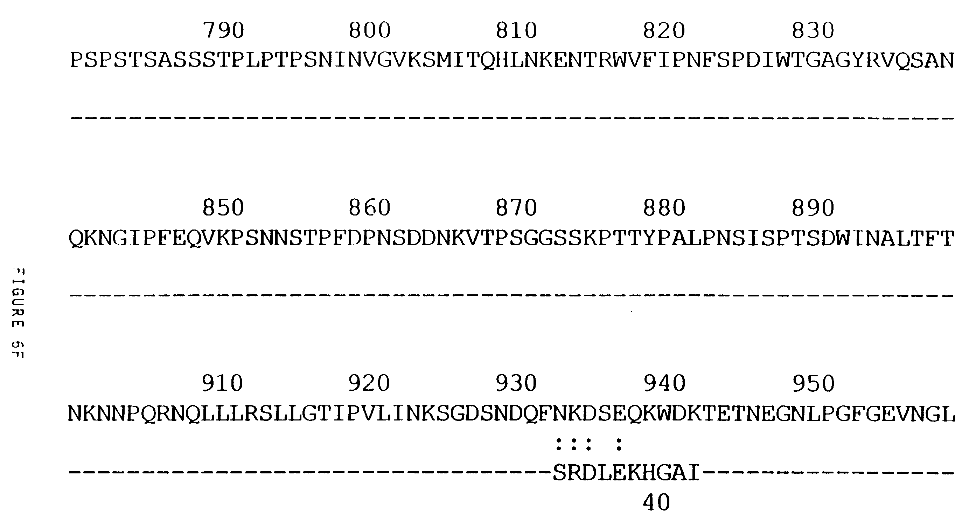

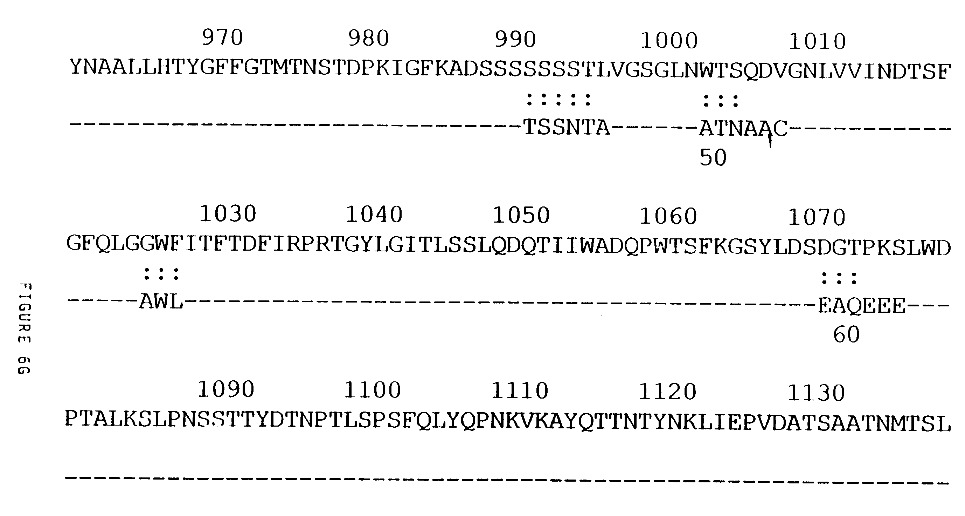

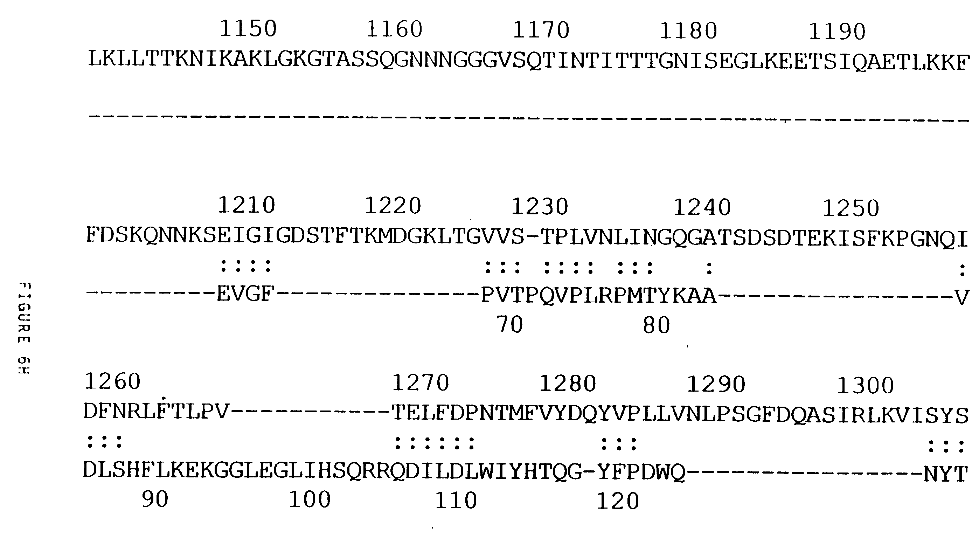

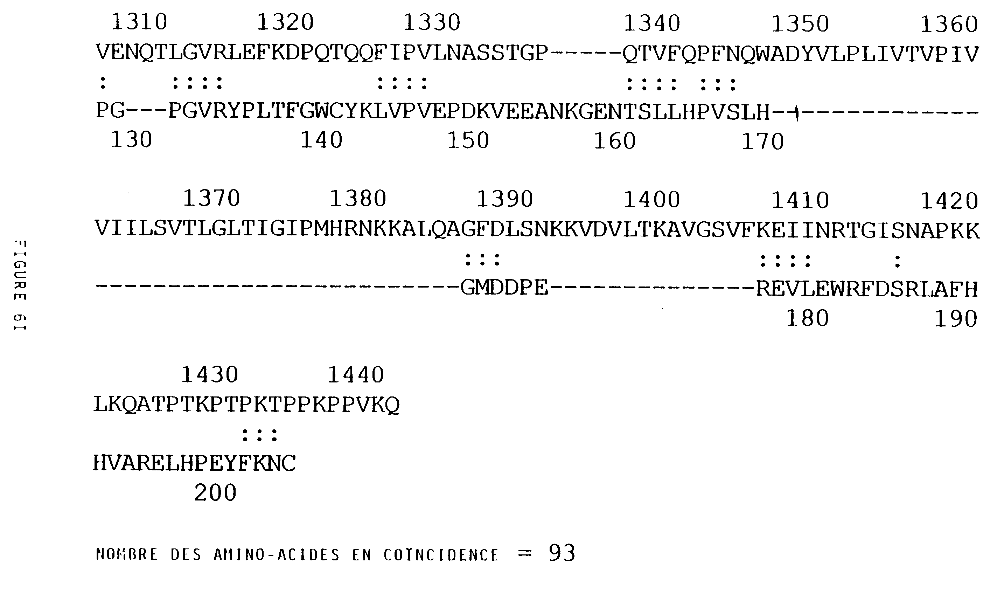

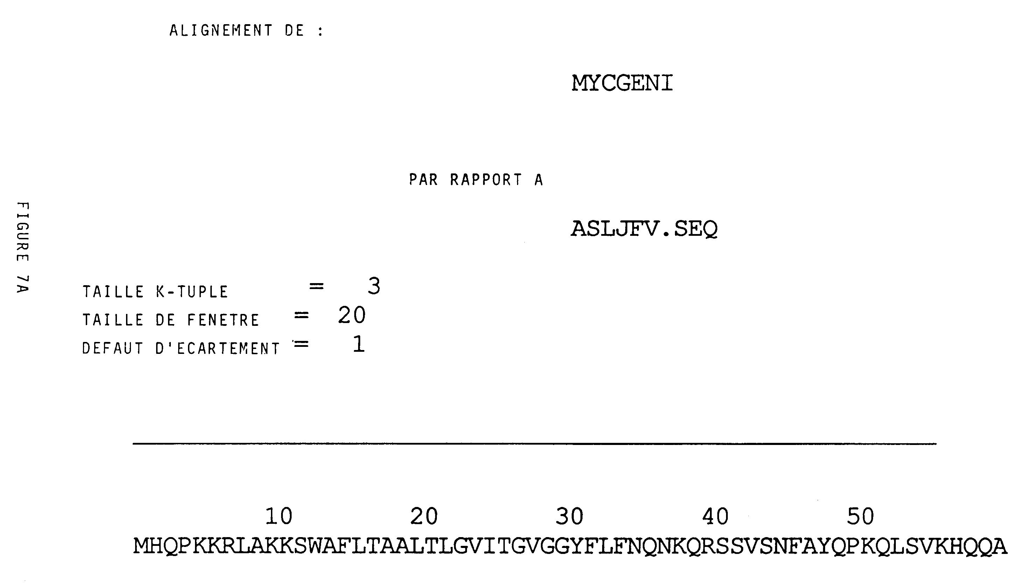

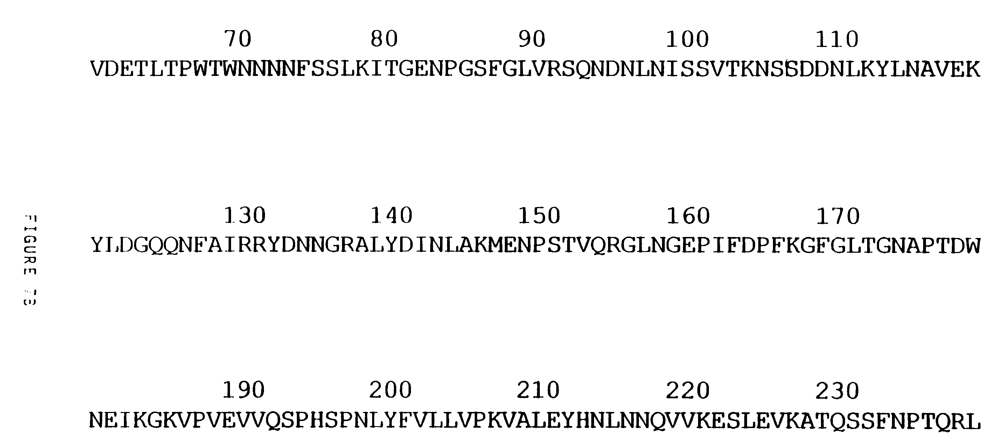

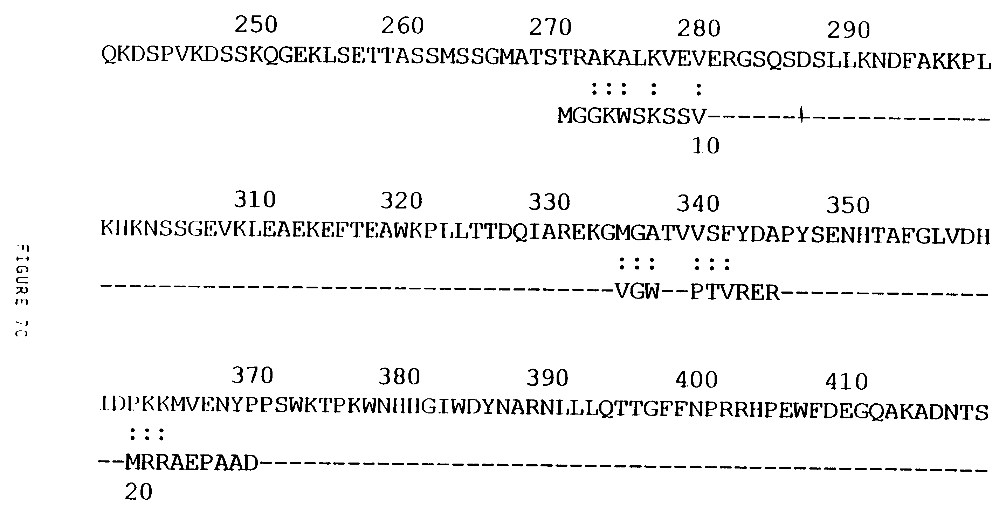

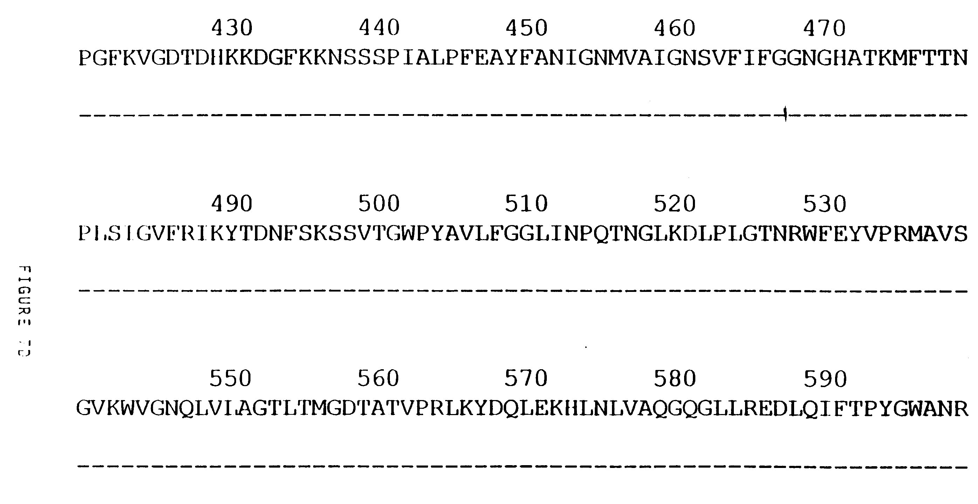

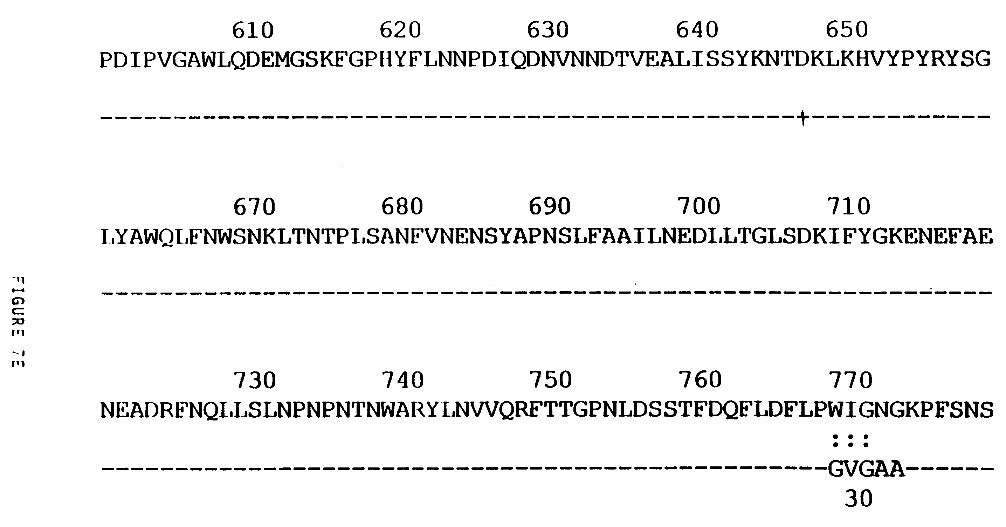

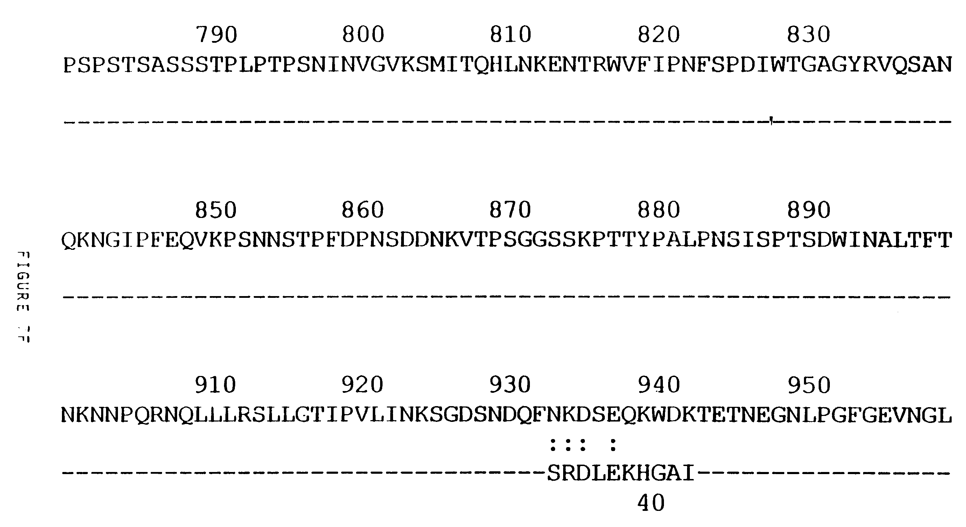

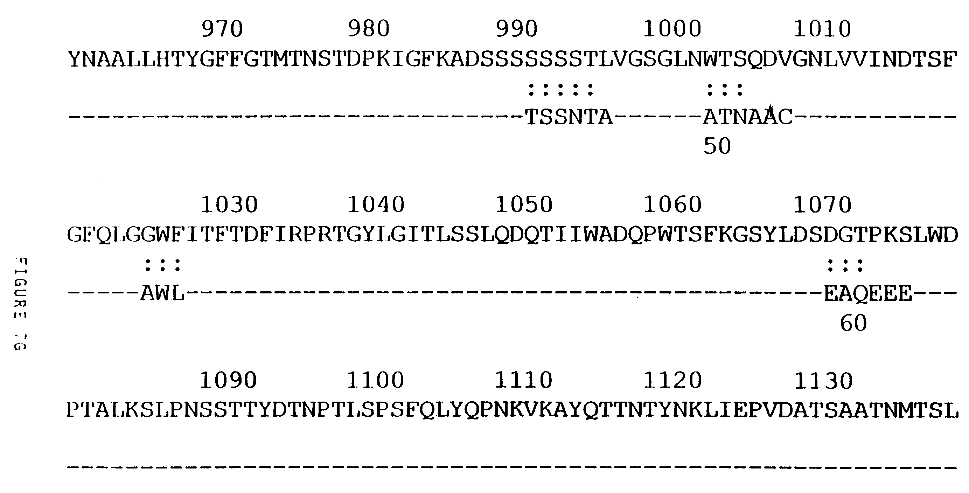

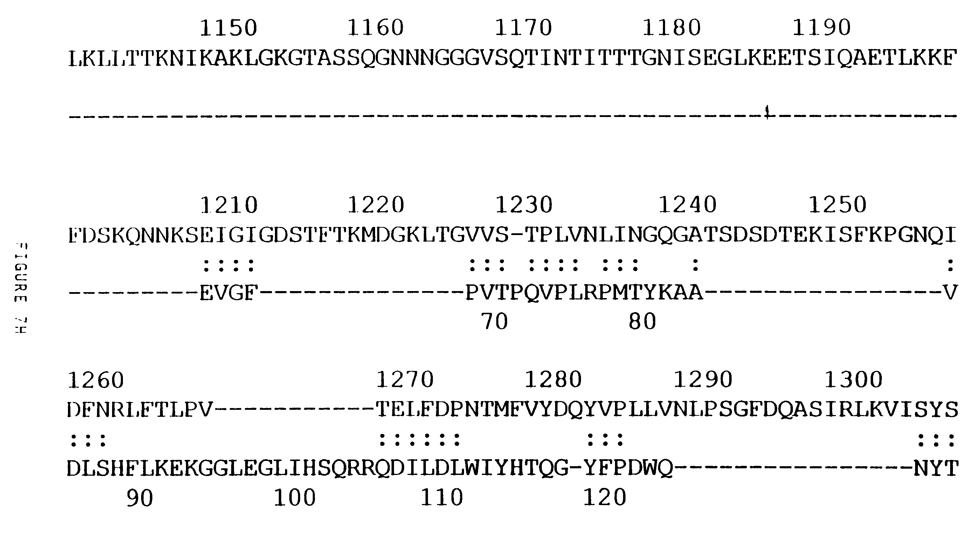

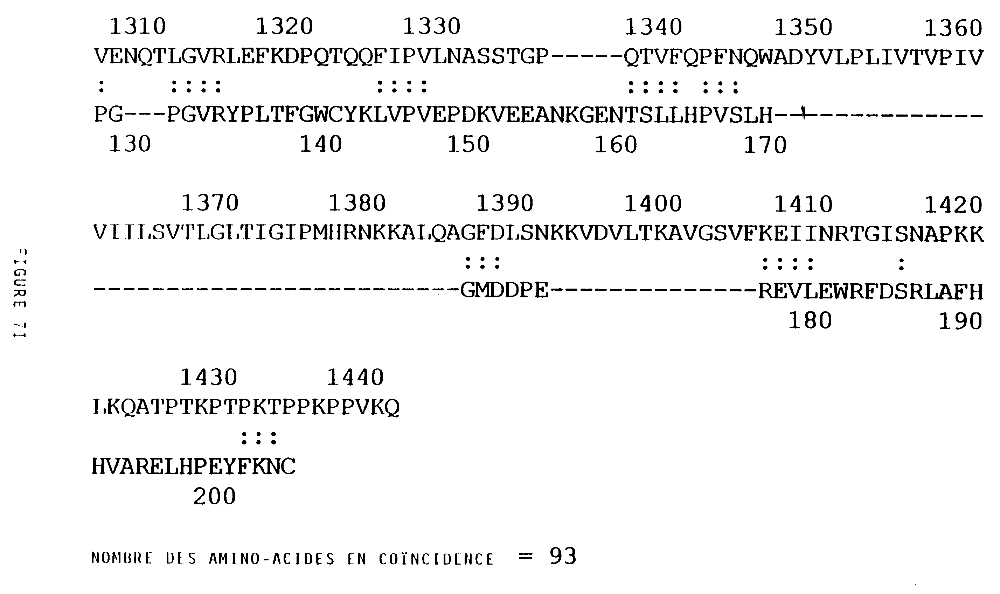

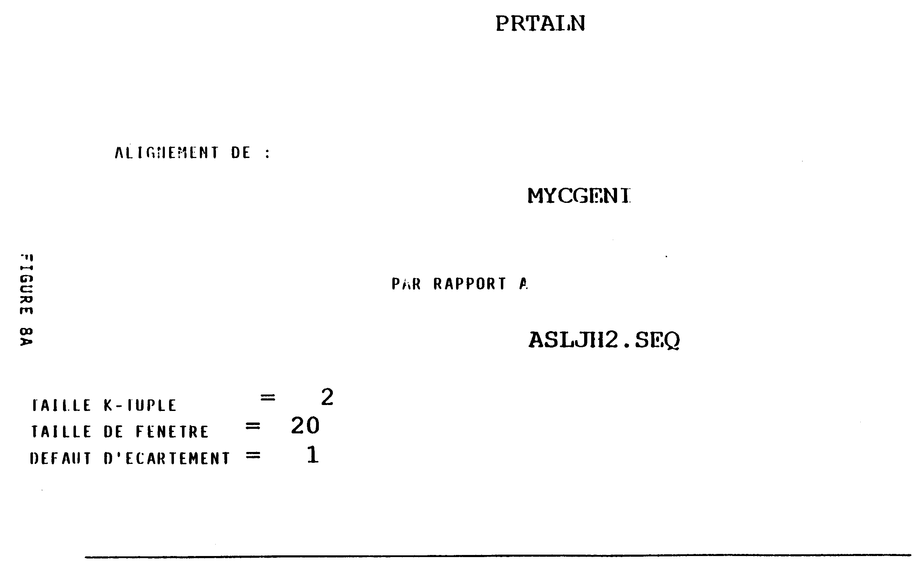

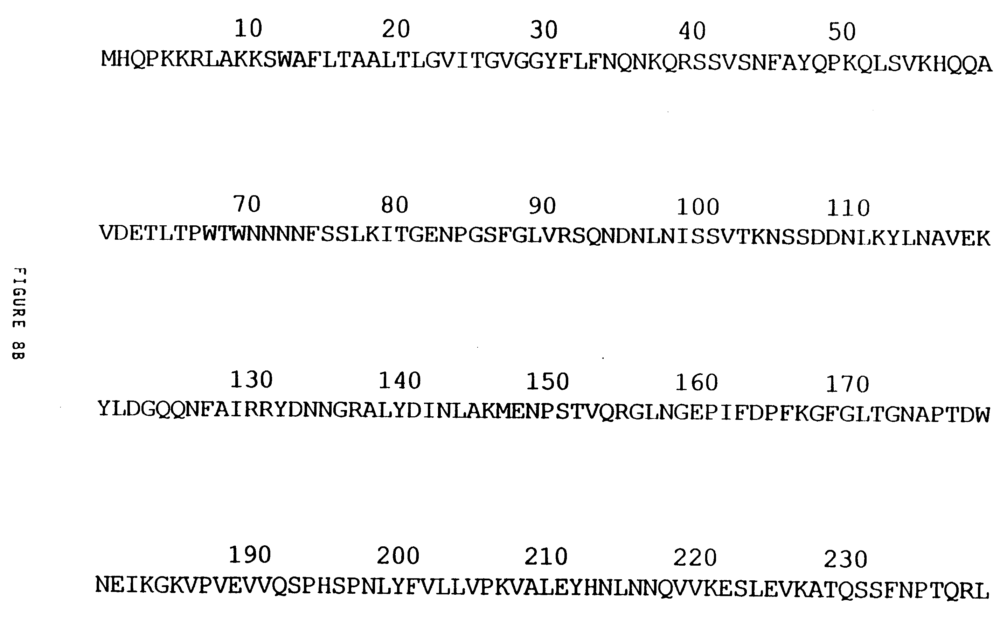

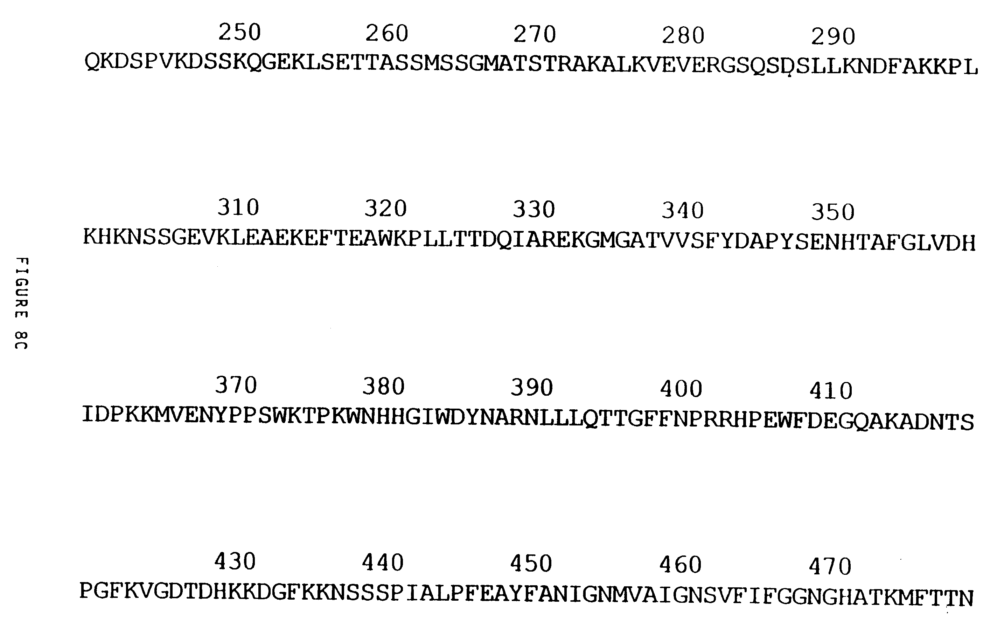

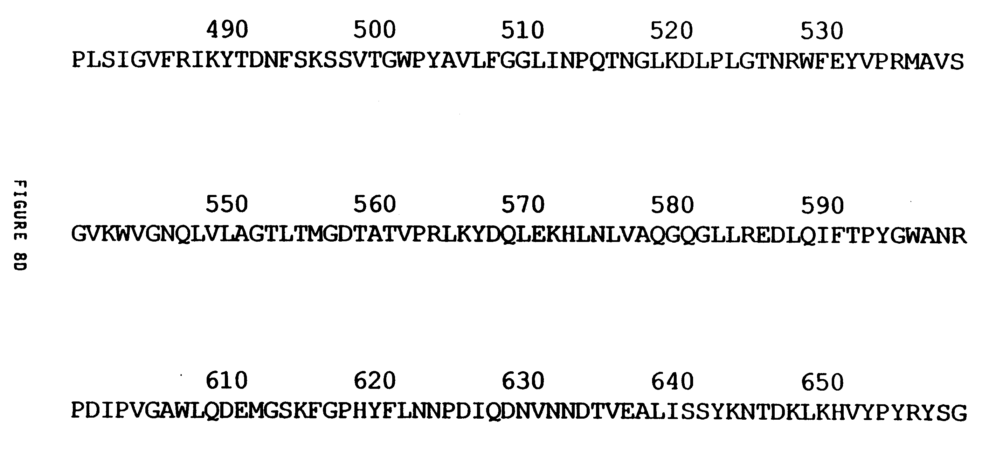

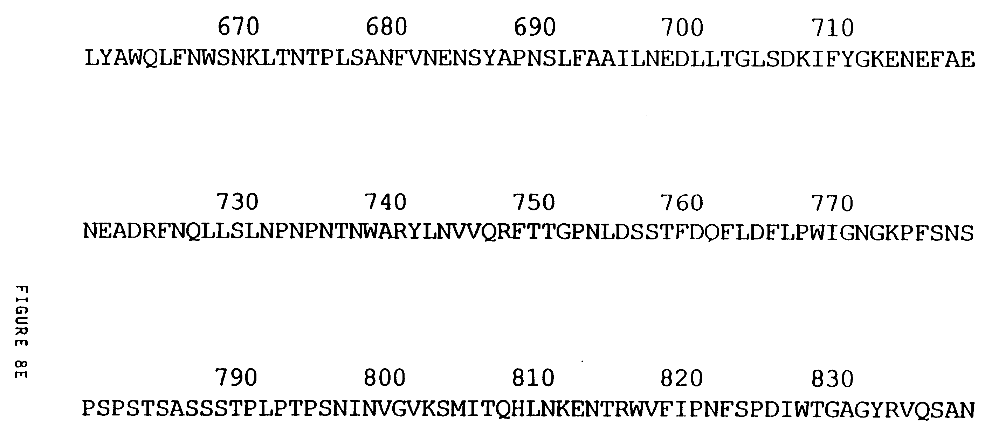

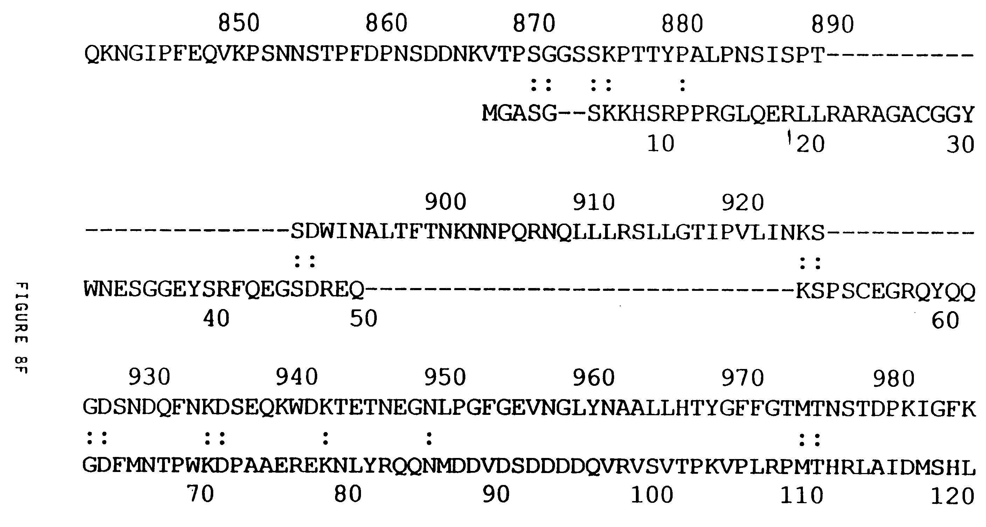

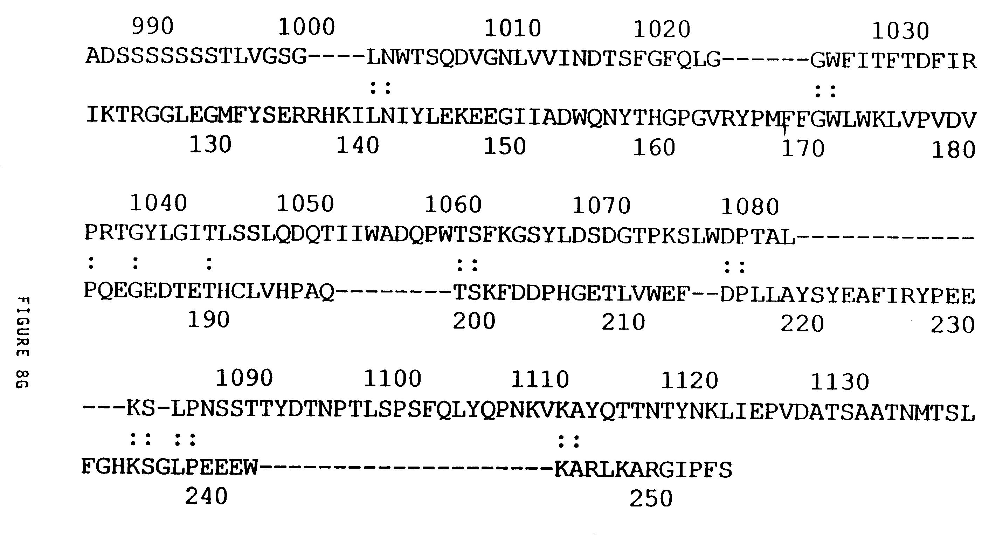

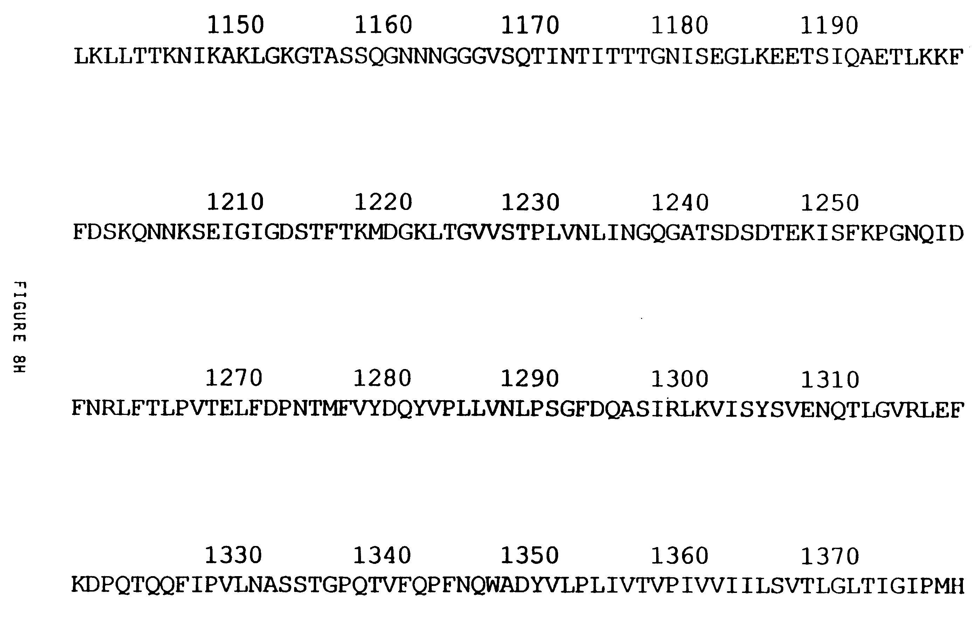

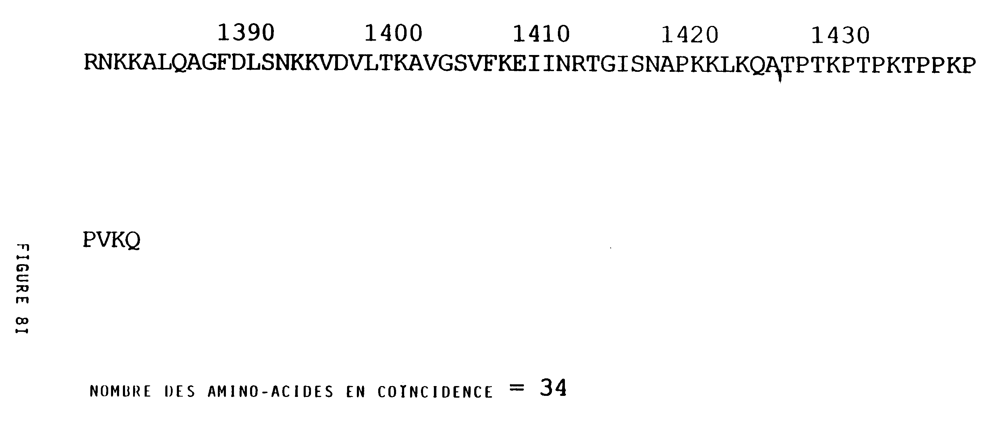

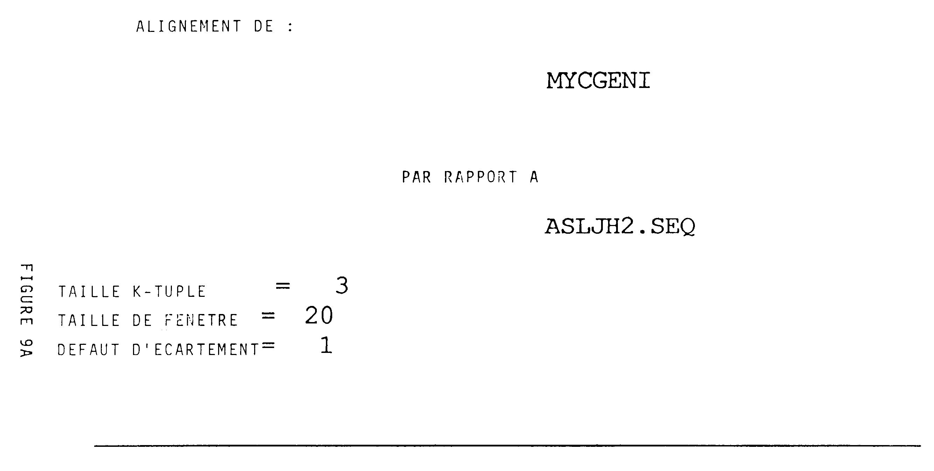

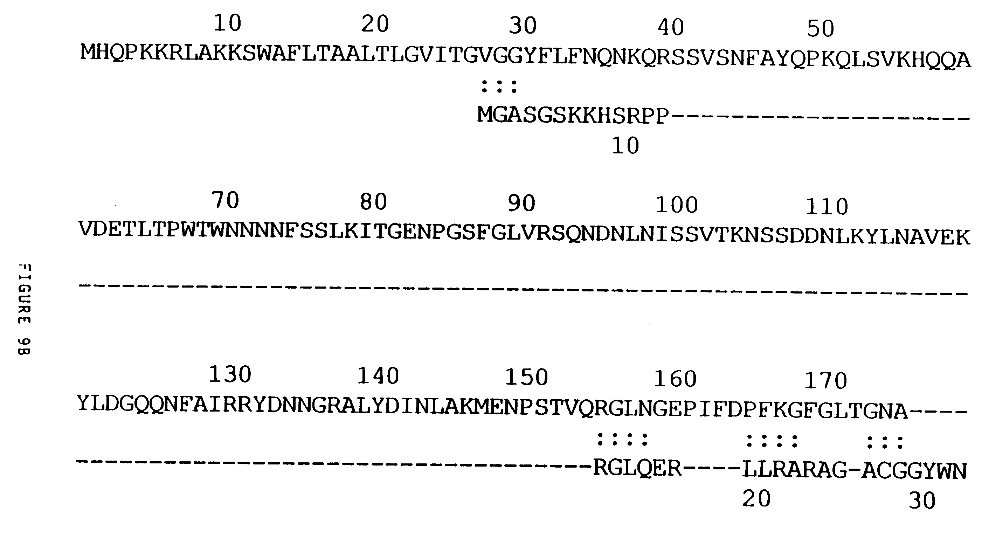

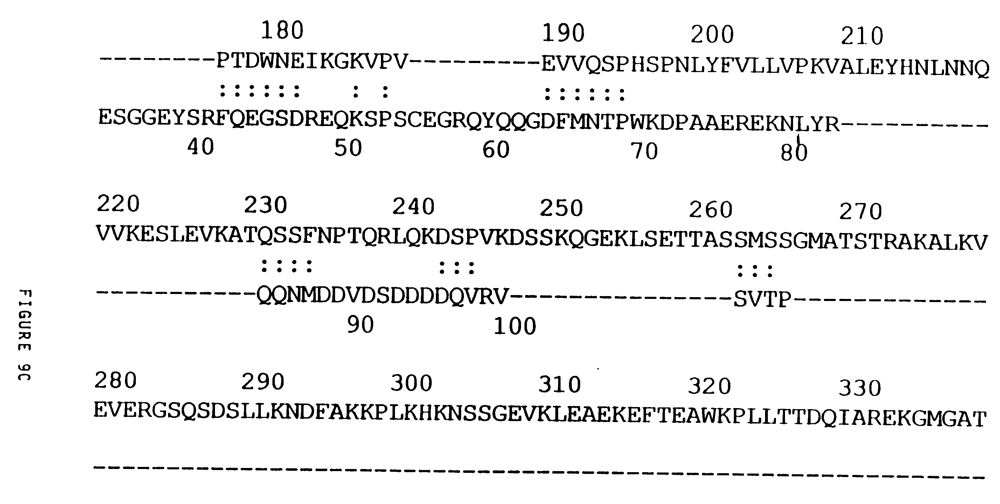

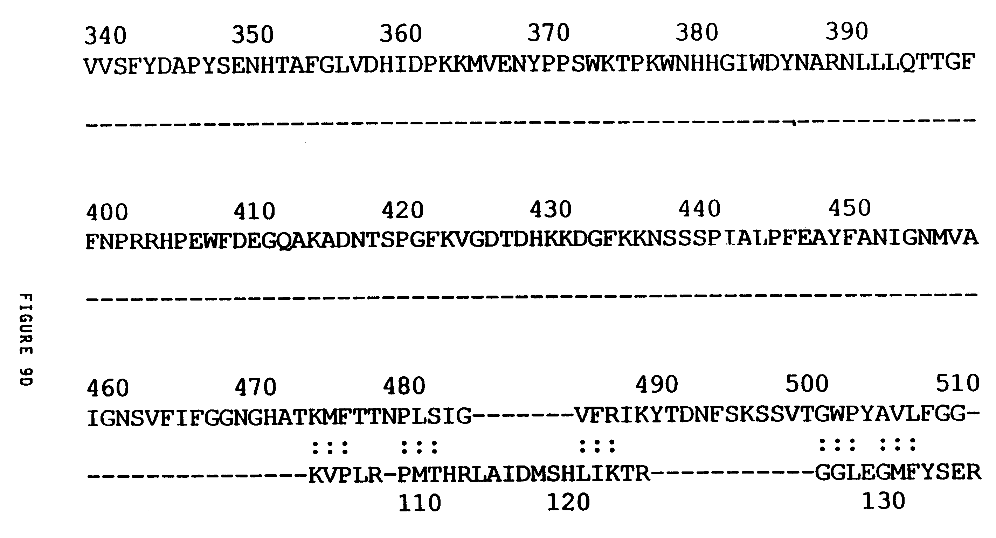

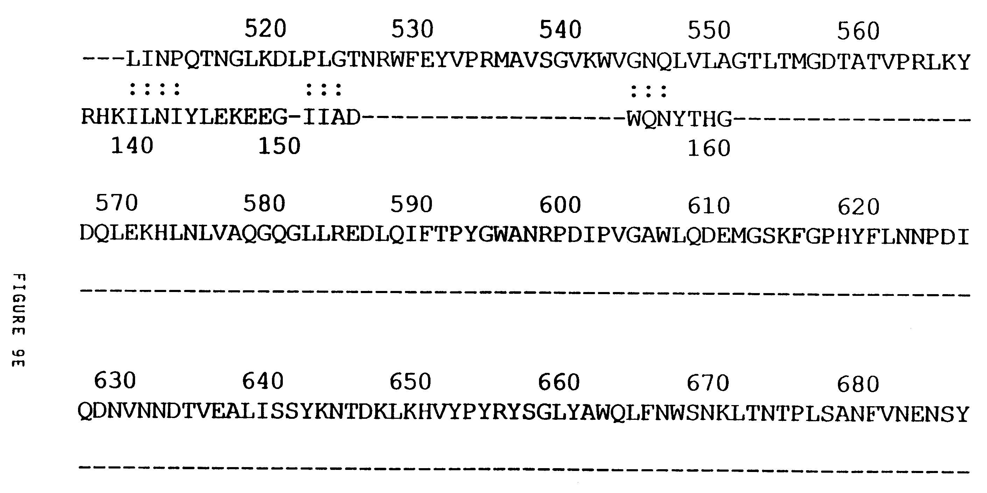

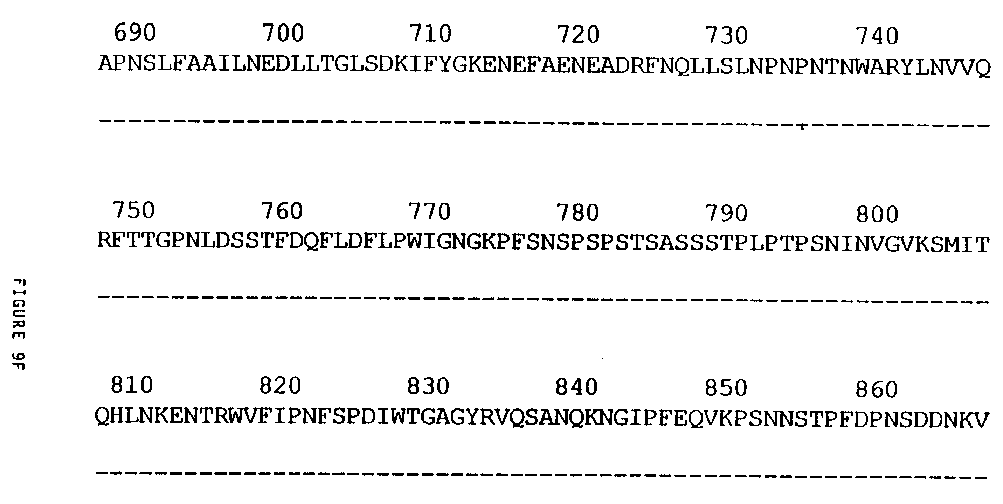

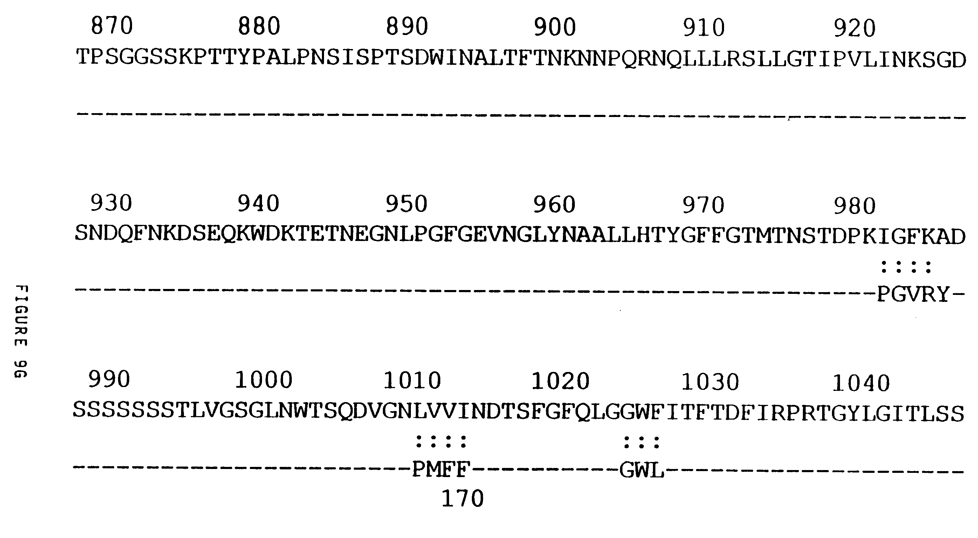

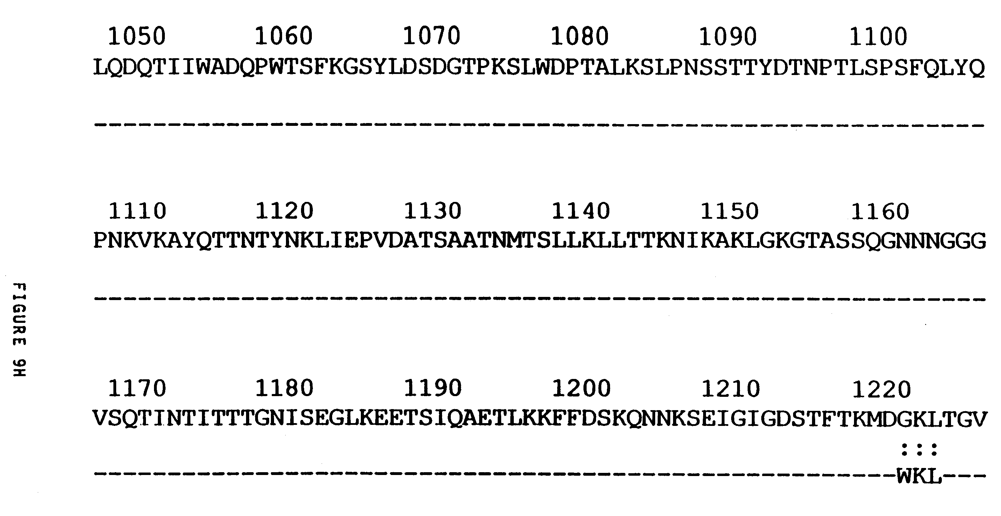

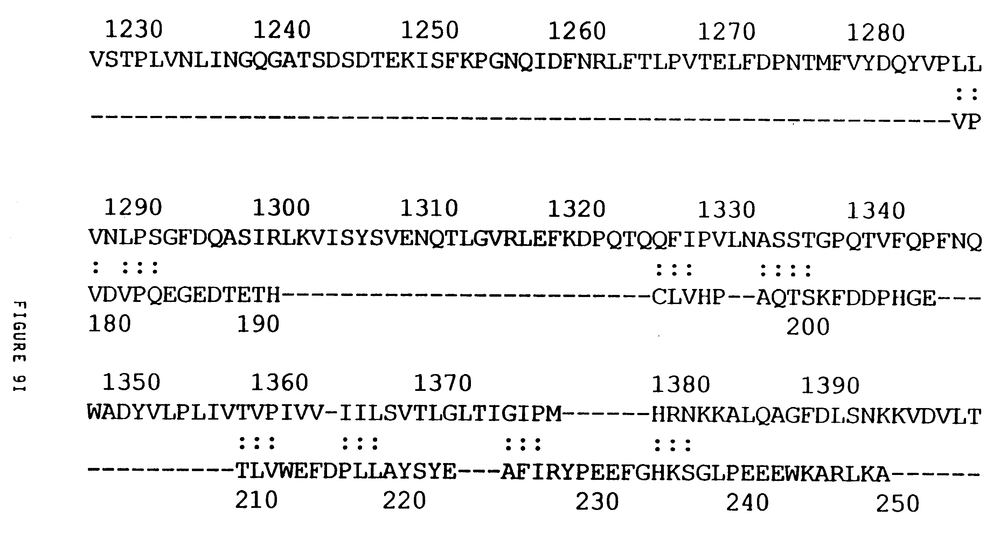

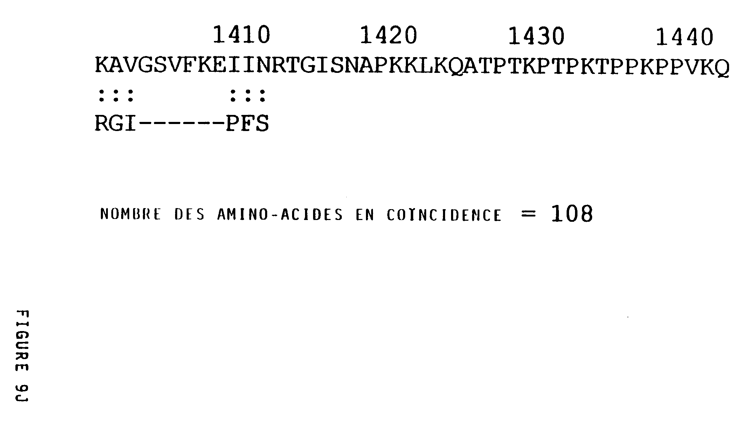

Les figures 2 à 9 regroupent les autres isologies et homologies obtenues pour HIV-1 et HIV-2.

### E) Homologies entre la p27 nef de HIV-1 et Mg Pa de M.genitalium

Une remarquable homologie de 90% existe pour une région de 9 acides aminés consécutifs conservés.

M.genitalium :     S K S S V T G W P
                    * * * * *   * * *
p 27 nef :          S K S S V V G W P

### F) Homologies entre la p55 gag de HIV-1 (BRU) et Mg Pa de M.genitalium

Aucune région d'homologie significative n'a pu être mise en évidence parmi les séquences étudiées.

L'invention concerne donc plus particulièrement les peptides ayant des séquences identiques à celles qui sont indiquées ci-dessus, qu'elles soient issues de HIV ou des mycoplasmes sus-indiqués. Elle concerne de même les peptides (plus longs ou plus courts) dont les séquences sont contenues dans celles des séquences les plus longues des susdits peptides, dès lors qu'ils correspondent aux susdites séquences mycoplasmiques, notamment celles issues de M. genitalium.

De même font encore partie de l'invention des peptides même encore plus courts, par exemple SKSSVTGWP dès lors qu'il sont eux-mêmes aptes
- soit (le cas échéant après leur couplage avec une molécule porteuse) à induire la production in vivo d'anticorps aptes à neutraliser la multiplication de retrovirus dans les cultures,
- soit à interférer eux-mêmes avec cette multiplication.

L'invention concerne encore les peptides qui se distinguent des précédents, par application des règles d'isologie dont il a été question plus haut, par exemple des peptides dans lesquels ceux des acides aminés présents qui appartiennent à des familles isologiques telles que définies plus haut peuvent être interchangés avec d'autres acides aminés appartenant respectivement aux mêmes familles isologiques.

Il résulte de ce qui précède - dans les limites des investigations qui ont été conduites à ce jour - que M.genitalium ou M.pirum ou de mycoplasmes apparentés sont une source préférée de séquences peptidiques utilisables pour les applications préférées de l'invention, notamment l'inhibition de la propagation de HIV dans les milieux biologiques qu'il sont susceptibles d'infecter.

Les peptides issus de protéines de mycoplasmes ou d'une autre source, dès lors qu'ils contiennent des séquences identiques ou semblables sont ceux qui induisent la synthèse d'anticorps inhibant in vitro l'infection à HIV quel que soit le degré d'homologie ou d'isologie à l'égard d'HIV.

L'invention concerne aussi les anticorps, polyclonaux ou monoclonaux, spécifiquement dirigés contre les susdits peptides. Des anticorps préférés sont ceux qui reconnaissent spécifiquement les séquences des régions d'adhésion des protéines adhésines de mycoplasme et en particulier p1 de M.pneumoniae, de M.genitalium, ou de M.pirum.

Des anticorps formés contre de tels peptides, notamment contre le peptide A ont inhibé à 90% l'infection, par la souche prototype HIV-1 (BRU), d'une culture de cellules mono-nucléées de sang périphérique en majorité des lymphocytes T activés par la phytohémagglutinine (PHA). En présence de ces anticorps aucune formation de syncitia n'est observée. Des résultats identiques ont été obtenus avec HIV-2 (ROD). Ceci est un résultat intéressant pour la compréhension de la pathogénèse de l'infection à HIV dans la mesure où la prévention de cette infection n'impliquerait pas seulement le HIV-1 et HIV-2 mais également un éventuel co-facteur ou un agent opportuniste tel qu'un mycoplasme spécifique qui "faciliterait" le cas échéant l'infection par les HIV.

De telles préparations d'anticorps, dirigées contre une séquence peptidique correspondant probablement au site d'interaction de Mycoplasma genitalium ou d'un autre mycoplasme, par exemple M.pirum, avec les cellules eucaryotes, inhibent l'infection de lymphocytes T activés ou des cellules de la lignée CEM par la souche protype du HIV-1, BRU (11).

La séquence complète de l'adhésine Mg Pa de M. genitalium est indiquée sur la ligne supérieure figurant aux figures 2 à 9.

Ces résultats sont le mieux expliqués par l'effet neutralisant de l'anticorps ainsi obtenu sur la fixation cellulaire d'un mycoplasme accompagnant cette souche virale. Cela signifierait que ce mycoplasme jouerait un rôle-clé dans le déclenchement de la réplication du virus.

### 1°/ Choix d'une séquence immunisante préférée

Peu de séquences protéiques de mycoplasmes ont été déterminées à partir des gènes clonés et celles qui sont connues révèlent une grande variabilité d'une espèce à l'autre.

Cependant pour au moins deux espèces parasites de l'homme, M.pneumoniae et M.genitalium, des protéines d'adhésion (adhésine) de masse moléculaire élevée ont été caractérisées (12) et leurs séquences publiées (6) (15).

La comparaison de ces séquences en acides aminés révèle une certaine homologie (50%), notamment au niveau de la séquence supposée être impliquée directement dans la fixation de M.pneumoniae à des eucaryotes (12).

Cette séquence est la suivante (peptide B) pour M.pneumoniae:
G I V R T P L A E L L D G E

Nous avons, après alignement, retrouvé une séquence homologue (50%) sur la séquence publiée de M.genitalium (peptide A) :

G I V R T P L A E L L D G E
*   *   * * *     *     *
G V V S T P L V N L I N G Q (peptide A)

Deux variantes du peptide A consistent en ce qu'elles comportent les séquences suivantes :
G V V S T P L V N L I N G
G V V S T P L V N L I N

Un épitope particulier consiste en la séquence :
T P L

Ce peptide fait lui-même partie de l'invention.

La conservation relative susdite suggère que les deux mycoplasmes, et peut-être d'autres espèces, utilisent cette séquence pour reconnaître une structure commune à la surface des cellules eucaryotes qu'ils parasitent.

Nous avons choisi préférentiellement la séquence de M.genitalium pour induire des anticorps spécifiques de cette séquence, du fait que ce mycoplasme est présent sur les muqueuses respiratoires, rectales et génitales (16), et que nous l'avons mis en évidence sur les globules rouges et les lymphocytes d'un malade atteint de SIDA. D'autres mycoplasmes ont été isolés du tractus intestinal chez l'homme.

Il a aussi été montré chez les patients immunodéprimés, qu'un mycoplasme, M.hominis, pouvait être isolé à partir d'organes, tissus et fluides différents. On se reportera par exemple à l'article de D. Mac Mahon et al. "Extragenital M.hominis infections in adults" publié en 1990, Vol. 89, p. 275 à 281 dans Am. Journal of Medicine.

Les protéines et peptides issus de ces différentes souches et qui répondent aux conditions qui ont été définies plus haut, entrent dans la définition des peptides selon l'invention. On peut en particulier se référer à l'article de GIEBEL J., BINDER A. et al "Isolation of Mycoplasmas from the intestine of rat, swine and man" (Zentralblatte für Bacteriologie, 1990, supplement N° 20, p. ).

A titre de contrôle, la séquence correspondant aux 25 acides aminés de l'extrêmité C terminale de la même protéine a été synthétisée (peptide C) :
A P K K L K Q A T P T K P T P K T P P K P P V K Q

### 2°/ Synthèse du peptide

La méthode développée par Merrifield (17) de synthèse en phase solide a été utilisée. L'assemblage des peptides a été mis en oeuvre de façon automatique sur un synthétiseur APPLIED BIOSYSTEMS modèle 430A.

Une partie (10 mg) a été couplée à une protéine porteuse, la "Keyhole limpet hemocyanin" (KLH, 10 mg) par la méthode à la glutaraldéhyde (18). L'efficacité de couplage était de 50 à 75%, d'après la détermination de la partie non couplée du peptide encore présent après passage sur une colonne de Séphadex G 25.

On peut également avoir recours (pour la production des peptides) à des techniques de recombinaison génétique, mettant par exemple en oeuvre des plasmides appropriés transfectant des hôtes cellulaires tels que bactéries, levures, cellules eucaryotes, etc... et contenant des séquences nucléotidiques codant pour les peptides selon l'invention, y inclus tout ou partie des séquences de protéine de mycoplasme les contenant ou d'autres séquences protéiques, dès lors que les produits d'expression correspondants induisent la production d'anticorps conservant leurs propriétés d'inhibition à l'égard d'une infection à HIV.

Comme on le verra plus loin, l'invention concerne également des associations ou combinaisons des peptides de l'invention ou d'autres peptides, notamment des peptides présentant une activité immunogène en ce qu'il induisent in vivo la production d'anticorps aptes à neutraliser des infections par HIV, notamment HIV-1 et HIV-2.

L' invention concerne plus particulièrement des peptides recombinants ou hybrides contenant des séquences correspondant à l'un et à l'autre des peptides sus-indiqués. Ces peptides recombinants ou hybrides sont notamment accessibles par la technique de recombinaison génétique dont le principe a été rappelé plus haut, en mettant en oeuvre les plasmides ou les vecteurs viraux par exemple le virus de la vaccine, le baculovirus, etc... appropriés, contenant alors les séquences nucléotidiques qui codent pour ces peptides recombinants ou hybrides et dont l'expression peut être effectuée dans des hôtes cellulaires eucaryotes y inclus la levure (Saccharomyces cerevisiae, Pichia pastoris etc...) ou dans des bactéries.

A titre d'exemple, un recombinant préféré est constitué par une séquence nucléotidique codant pour la région V₃ de la gp 120 en particulier le peptide dont les acides aminés portent les numéros 307 à 321, du HIV-1 (Rusche J. et al., PNAS, 1988, 85, p. 3198-3202) associée à la séquence de la protéine adhésine de M.genitalium dont la séquence du gène a été décrite dans Gene, 1989, **82**, p.259-267 (Inamine et al) ou de l'adhésine de M.pirum ou de Mycoplasmes apparentés et de préférence la séquence nucléotidique correspondant à la séquence du peptide A. Un tel hybride exprimé dans un hôte cellulaire eucaryote y compris les levures par exemple Pichia, Kluyveromyces ou un hôte procaryote permet l'obtention d'une protéine ou d'un peptide hybride qui peut constituer l'un des composants d'une composition immunogène induisant des anticorps qui inhibent une infection à HIV in vitro. On peut se référer pour le choix des séquences d'HIV1 ou d'HIV2 exprimant une protéine à l'article de GUYADER et al Nature, 1987, 326, p. 662-669.

Ces mêmes peptides hybrides peuvent être utilisés à des fins de diagnostic in vitro. La méthodologie utilisée est celle décrite dans le brevet US n° 4.839.288.

### 3°/ Préparation et caractérisation des anticorps :

Des lapins de Nouvelle Zélande ont été immunisés comme suit : 200 µg du peptide couplé à la KLH mélangé avec un volume égal d'adjuvant complet de Freund sont injectés par voie intradermique au jour O. Ce protocole a été ensuite répété par voie sous-cutanée en présence d'adjuvant incomplet de Freund aux jours 30, 60 et 90.

Les animaux sont saignés pour obtenir le sérum une semaine après chaque injection. Du sérum des mêmes animaux a été conservé avant le début de l'immunisation, à titre de témoins des effets biologiques des sérums.

Dans le cas où le peptide A est utilisé comme agent immunogène couplé à la KLH, les anticorps ainsi obtenus reconnaissent spécifiquement le peptide immunisant et aussi, en utilisant la technique du Western-Blot, une protéine de 140 KDa dans un extrait solubilisé de M.genitalium (G37) par une solution aqueuse de 1% (poids/volume) de sodium dodécyl sulfate. La solution utilisée est M.genitalium G37 (ATCC N° 33530).

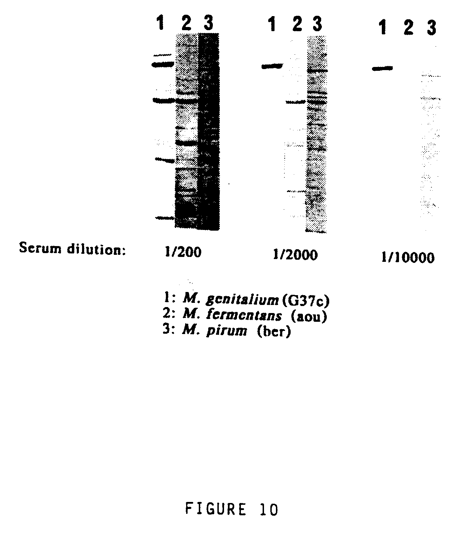

La protéine de 140KDa correspond à l'adhésine qui a été décrite dans la littérature et est reconnue par l'antisérum jusgu'à une dilution d'au moins 1/100.000 de ce dernier (Figure 10).

A une dilution plus faible (1/2000), l'anticorps anti-peptide A reconnait également une protéine de 130 KDa contenue dans un extrait lysé de Mycoplasma pirum (souche Ber) (déposée à la C.N.C.M. le 3 mai 1990 sous le numéro I-950 et à la N.C.I.N.B sous le N° 40283 le 17 mai 1990), un mycoplasme que l'on trouve également fréquemment chez les malades atteints du SIDA, et qui pourrait donc également contaminer les souches de HIV.

Les mesures de poids moléculaires des protéines susdites (obtenues à partir de lysats de mycoplasmes concervés dans du SDS) (dodecyl sulfate de sodium) à 1% ont été mesurées par comparaison de leurs distances de migration respectives avec celles de six protéines de référence, dans un gel d'électrophorèse, ayant des poids moléculaires indiquées ci-après (coffret d'analyse ou "kit" produit par Bio-Rad) :

| | |
|---|---|
| Lysozyme | 14.400 |
| Inhibiteur de trypsine de soja | 21.500 |
| Anhydrase carbonique | 31,000 |
| Ovalbumine | 45.000 |
| Serum albumine bovine | 66.200 |
| Phosphorylase B | 92.500 |

Ces mesures sont faites avec une précision de + ou - 10%. La protéine de 130 KDa peut être utilisée pour réactif constituant l'un des éléments d'un coffret pour un test ELISA ou un test RIA ou encore comme réactif après marquage par un marqueur fluorescent par exemple. La protéine P130 KDa de M. pirum peut être également utilisée comme agent immunogène pour induire la synthèse d'anticorps actifs contre une infection à mycoplasme.

Des réactions antigéniques plus faibles sont observées dans les mêmes conditions avec le même anticorps anti peptide A, avec des protéines de masse moléculaire inférieures à 65 KDa, en particulier avec trois protéines de masse moléculaire de 64 KDa, 45 et 42 KDa de M. pirum.

On trouve également fréquemment, chez les malades atteints du SIDA, les anticorps dirigés contre la susdite protéine de 130 KDa, issue de M.pirum, ou reconnaissant des protéines ayant des propriétés antigéniques semblables et qui pourrait donc également contaminer les souches de HIV isolées des malades.

Les méthodes utilisées pour la préparation des peptides sélectionnés ou des anticorps correspondants peuvent naturellement être transposées à la fabrication d'autres peptides ou anticorps entrant dans le champs de la présente invention. Pour les anticorps monoclonaux, on utilise la méthode décrite par Köhler et Milstein (NATURE, 1975, 256, pages 495-497).

### 4°/ Effet des anticorps sur l'infection par HIV :

Des lymphocytes de donneurs HIV négatifs sont activés par la phytohémaglutinine pendant trois jours et cultivés en présence de protéine d'interleukine 2 en milieu RPMI 1640 avec 10% de sérum de veau fetal, 100 U/ml de pénicilline et 50 U/ml de streptomycine. Ils sont infectés par la souche prototype HIV-1 LAV_{BRU} à raison de 10⁴ cpm d'activité transcriptase inverse par 10⁶ cellules. La souche HIV-1 BRU est un échantillon de la souche CNCM I-232.

D'autres flacons de la même culture sont infectés avec la même suspension virale, préalablement incubée une heure à 37°C avec différentes concentrations d'antisérums dilués au 1/50ème ou au 1/200ème de lapins immunisés contre les peptides de l'adhésine selon le procédé décrit supra.

Ils ont été injectés à des lapins après couplage avec la KLH et adjonction d'adjuvant complet de Freund. Les anticorps obtenus ont été testés selon la technique décrite par Szmelcman et al (8) pour évaluer leurs effets, après mise en contact avec les lymphocytes T en culture puis infectés par la souche prototype HIV-1 (BRU). Le dosage de la transcriptase inverse et le dosage de la production de p25 ont été effectués pour déterminer la production du virus.

La production de HIV par les cultures est suivie par l'activité transcriptase inverse et celle de l'antigène p25 est mesurée par ELISA (Diagnostic Pasteur) dans les surnageants de culture. Cette production est aussi appréciée par l'effet cytopathogène (formation de syncitias et lyse des cellules isolées) induit par le virus.

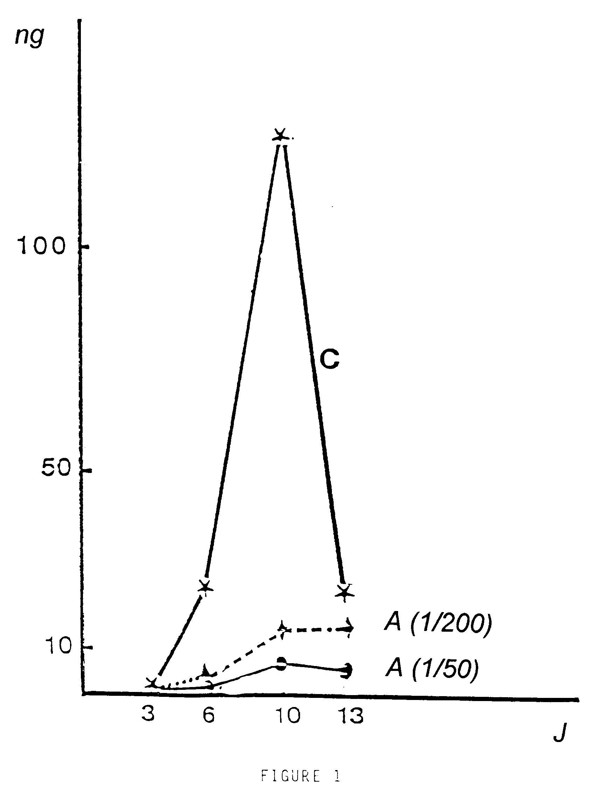

On constate que la production de p25, et par conséquent de virus est inhibée à plus de 90% par une dilution au 1/50^{ème} de l'antisérum contre le peptide A. Une moindre inhibition, mais encore notable, est obtenue au 1/200^{ème}. Les variations des taux d'inhibition de la quantité de p25 produite (p25) en ng sur l'axe des ordonnées, en fonction du temps (en jours (J) sur l'axe des abscisses) sont illustrées par les courbes de la figure 1. Les mesures ont été faites en présence d'anticorps contre le peptide A (aux dilutions de 1/50^{ème} et 1/200^{ème}) et du peptide C.

Les sérums de lapins prélevés avant immunisation n'ont pas d'effet, pas plus que les sérums des lapins immunisés contre le peptide C de la partie C-terminale de l'adhésine.

La spécificité de l'inhibition est montrée par le fait qu'elle est réduite par la préincubation du sérum anti-peptide A avec le peptide A en excès, l'inhibition résiduelle étant liée au peptide lui-même (voir plus loin).

L'inhibition par l'antisérum anti-peptide A est observée quand il est ajouté au moment de l'infection des lymphocytes T normaux par la préparation virale. Par "préparation virale" on entend un surnageant brut de culture de cellules lymphocytaires infectées par le virus HIV, ce surnageant pouvant contenir des mycoplasmes. Mais l'inhibition peut aussi être observée quand la préparation virale est préincubée pendant 1 heure à 37°C avec l'antisérum anti-peptide A, puis est ajoutée aux cellules pour l'infection. Dans ce cas, il n'est pas nécessaire, pendant la durée de l'expérience, de rajouter par la suite de l'antisérum au milieu de culture, pour obtenir l'effet d'inhibition maximum.

Des résultats similaires ont été obtenus avec la lignée cellulaire CEM clone 13, permissive à la souche HIV-1 BRU. Ces cellules sont préalablement propagées pendant trois semaines en présence d'antibiotiques ayant un large spectre d'action sur les mycoplasmes (MRA) avant d'être infectées par le virus, de façon à réduire la contamination endogène de cette lignée par des mycoplasmes.

On observe avec la lignée CEM infectée par une préparation virale d'HIV-1 (BRU) ou HIV-2 (ROD) traitée à l'antisérum anti-peptide A un retard dans la courbe de production du virus, mais pas une suppression totale de cette production, ainsi qu'un retard dans l'effet cytopathogène du virus. Cet effet cytopathogène se caractérise par la formation de grappes cellulaires, puis de syncitia ; on peut penser qu'il se produit secondairement une propagation de l'infection par contacts intercellulaires, ce mode d'infection n'étant plus sensible à l'antisérum anti-peptide A.

Une inhibition similaire par l'antisérum a été observée sur la souche HIV-2 ROD propagée sur les cellules CEM (figure 1 bis).

### 5°/ Effet du peptide A lui-même :

Si l'antisérum contre le peptide de A (ou peptide modèle du site de fixation du mycoplasme (Mycoplasma Binding Site Peptide)) inhibe la fixation aux cellules du mycoplasme contaminant le virus dans la préparation virale, il s'ensuit qu'on pourrait également neutraliser cette fixation par le peptide lui-même présent au moment où la préparation virus-mycoplasme est ajoutée aux cellules. Cette hypothèse a été vérifiée et on observe une inhibition de l'infectiosité du virus par le peptide à des concentrations relativement élevées (de l'ordre de 500 µg/ml).

En conclusion, nos résultats confirment qu'un mycoplasme de type M.genitalium ou M.pirum relativement proches de ces derniers, est impliqué dans une phase précoce de l'infection HIV-1_{BRU} (fixation, pénétration ou début de l'expression génomique dans les cellules T).

On peut donc envisager l'application de la présente invention à la préparation de compositions immunogènes, de vaccins ou de médicaments directement actifs, à la mise au point de nouvelles thérapeutiques, et à des méthodes diagnostiques :
1. Vaccination par le peptide A ou d'autres peptides efficaces conformes à l'invention, de sujets HIV-séronégatifs ou HIV séropositifs asymptomiques par le peptide A ou des peptides contenant la même séquence ou contenant des séquences permettant l'induction in vivo d'anticorps spécifiques ayant des propriétés identiques ou semblables contre l'infection à HIV, le cas échéant en présence d'un adjuvant approprié à l'homme, tel l'hydroxyde d'aluminium.
   Cette vaccination serait protectrice contre l'infection par HIV chez les sujets HIV-séronégatifs et protectrice de l'évolution vers le SIDA chez les sujets séropositifs asymptomatiques.
   Le cas échéant, les peptides contenus dans ces compositions vaccinantes sont eux-mêmes greffés sur des protéines ou polypeptides, porteurs aptes à renforcer l'immunogenicité recherchée desdits peptides.
   L'invention s'étend également à l'utilisation, en vaccination, des peptides selon l'invention, en association ou en combinaison avec un vaccin HIV distinct pour augmenter le pouvoir protecteur de ce dernier.
   L'invention concerne donc également des compositions immunogènes, plus particulièrement vaccinantes, à base de l'un au moins des peptides selon l'invention, soit pris seul, soit en combinaison avec des peptides immunogènes, plus particulièrement vaccinants, issus de HIV, ou avec des peptides qui leur sont apparentés (voir demandes de brevet européen rendues publiques au nom de l'lnstitut Pasteur, le cas échéant avec d'autres co-déposants, par exemple les demandes européennes publiées sous les N° EP 201540 (N° de dépôt 85.905513.9), et EP 283327 (N° de dépôt 88.400084.5). Un peptide issu de la glycoprotéine d'enveloppe gp 120 décrit par Rusche et al (PNAS 1988, 85, p 3198) est l'un des peptides préférés issus de HIV.
   On peut également utiliser comme peptide de HIV selon l'invention, la glycoprotéine d'enveloppe gp 120 ou des fragments de celle-ci dont l'activité a été décrite par BERMAN et al dans NATURE, 1990, Volume 345, page 622 à 625 (Protection of chimpanzees from infection by HIV-1 after vaccination with recombinant glycoprotein gp 120 but not gp 160) (Protection du chimpanzee contre l'infection par HIV) par vaccination avec une protéine recombinante gp 120, mais non gp 160). Ces compositions contiennent également, le cas échéant, les véhicules appropriés aux modes d'administration choisis, notamment par voie parentérale. On utilisera des doses immunogènes de peptide comprises entre 3 µg/kg et 10 µg/kg d'hôte recevant ces doses (Anderson, J. Inf. Dis., 1989, 160).
2. Thérapeutique chez les sujets immunodéprimés (en état de SIDA), soit par injection intraveineuse du peptide lui-même, soit par administration d'anticorps préalablement formés contre des peptides conformes a l'invention, en particulier des anticorps anti-peptide A préparés chez l'animal (immunothérapie passive) ou des anticorps monoclonaux. L'utilisation de fragments actifs purifiés, par exemple des fragments FAB de ces anticorps sera préconisée pour éviter les accidents d'hypersensibilité.
3. Diagnostic :
   Mesure chez les malades et les personnes asymptomatiques du taux d'anticorps contre le peptide A ou autres peptides conformes à l'invention, originaires de mycoplasmes et susceptibles d'induire des anticorps inhibant ou diminuant une infection à HIV in vitro ou in vivo.
   Application au diagnostic et au suivi de l'effet de médicaments actifs sur les mycoplasmes et dans le SIDA.

Les figures 2 à 9 qui suivent fournissent à la fois les séquences d'acides aminés de protéines issues de M. genitalium et de différentes protéines issues soit de HIV-1 ou de HIV-2.

Ces figures permettent d'identifier les peptides de l'invention qui, par application des règles d'isologies et d'homologies définies plus haut, font partie de M. genitalium et qui présentent des isologies, voire même des homologies, avec des régions correspondantes des différentes protéines de HIV-1 et HIV-2.

Plus particulièrement on note les séquences des acides aminés de la protéine p1 de M. genitalium qui se trouvent alignées sur :
Fig. 2A à 2J :
   la séquence en acides aminés de l'enveloppe (alignement par homologies directes) de HIV-2 ROD
Fig. 3A à 3K :
   la séquence de la protéine de l'enveloppe de HIV-2 ROD
Fig. 4A à 4K :
   a séquence de la protéine d'enveloppe de HIV-1 BRU
Fig. 5A à 5J :
   la protéine d'enveloppe de HIV-1 BRU
Fig. 6A à 6I :
   la séquence de p27 nef de HIV-1 BRU
Fig. 7A à 7I :
   la protéine p27 nef de HIV-1 BRU
Fig. 8A à 8I :
   des séquences de p31 nef de HIV-2 (ROD)
Fig. 9A à 9J :
   des séquences de p31 nef HIV-2 ROD.

l'attention est encore attirée :
- sur la figure 1bis illustrant l'inhibition dans le temps (en jours sur l'axe des abscisses) de la production de reverse transcriptase virale (activité en ordonnées) dans des lymphocytes T placas en présence de HIV-2 et d'anti-peptides de A, par comparaison aux observations faites sur une culture « contrôle », en l'absence d'anticorps anti-peptide
- sur la figure 10 montrant les bandes d'électrophorèses observées aux dilutions indiquées dans des essais réalisés par analyse dite de « Western blot » et effectuées sur des sérums. On voit apparaître la présence d'une bande caractéristique du site de fixation du peptide A dans les essais mettant en oeuvre M. genitalium.

Dans le but de confirmer les résultats obtenus avec les anticorps de lapins immunisés avec le peptide A couplé à la KLH, le même peptide ainsi couplé a été utilisé pour immuniser 2 chevaux selon le calendrier suivant :

| **CALENDRIER D'INJECTION ET DE PRELEVEMENTS** | | |
|---|---|---|
| Période | Injection | Prélèvement |
| J0 | 1mg Pep P/CFA vol à vol. | 450 ml de sang stérile |
| J30 | 1mg Pep P/IFA vol à vol. | 450 ml de sang stérile |
| J40 | 1mg Pep P/IFA vol à vol. | 450 ml de sang stérile |
| J60 | 1mg Pep P/IFA vol à vol. | 450 ml de sang stérile |
| J70 | 1mg Pep P/IFA vol à vol. | 450 ml de sang stérile |
| J90 | 1mg Pep P/IFA vol à vol. | 450 ml de sang stérile |
| J100 | 1mg Pep P/IFA vol à vol. | 450 ml de sang stérile |
| J120 | 1mg Pep P/IFA vol à vol. | 450 ml de sang stérile |
| J140 | 1mg Pep P/IFA vol à vol. | 450 ml de sang stérile |
| J30 | 1mg Pep P/IFA vol à vol. | 450 ml de sang stérile |
| J30 | 1mg Pep P/IFA vol à vol. | 450 ml de sang stérile |

Les sérums déjà prélevés ont montré une forte immunoréactivité contre le peptide en ELISA (titre des sérums de l'ordre de 1/10⁶).

Des immunoglobulines sont en cours de purification afin de préparer les fragments Fab correspondant. Le sérum total, les immunoglobulines purifiées ainsi que les fragments Fab seront testés pour leur capacité à inhiber in vitro l'infection des cellules CD₄⁺ par le virus HIV.

### BIBLIOGRAPHIE

1. Lo S.C. et al.
   A.M.J. Trop. Med 1989, 41 (5)n 601-616
2. Saillard et al.
   Res. Virol., 1990, 141, 385-395
3. Chowohury I.H. et al.
   The Lancet 1990, Vol 336, 247-248
4. Lemaitre M et al.
   Res. Virol, 1990, 141, 5-16
5. Poch O. et al.
   The EMBO Journal, 1989, vol 8, N° 12, 3867-3874
6. Inamine et al.
   Gene, 1988, 64, 217-229.
7. Morrison-Plummer J. et al.
   Infection and Immunity, 1987, vol. 55, N° 1, 49-56
8. Scmelcman S. et al.
   J. of AIDS, 1990, vol. 3, N° 9, p. 859-887
9. Wibur W.J., Lipman D.
   Protc. Natl. Acad. Sci. USA, 1984, 80, 726-730
10. Lipman D., Pearson W.
   Science, 1985, vol. 227, 1435-1441
11. Barre-Sinoussi F. et al.
   Science, 1983, 220, 868-871.
12. Dallo S.F. et al.
   J. Exp. Med., 1988, 167, 718-723.
13. Wain Hobson et al.
   Cell, 1985, 40, 9-17.
14. Freed E.O. et al.
   Bull. Inst. Past. 1990, 88, p. 73-110
15. Dallo S.F.et al.
   Infection and Immunity, 1989, 57, 1059-1065.
16. Hooton T.M.
   Lancet, 1988, 1:266-268.
17. Merrifield R.B.
   J. AM. Chem. Soc., 1963, 85, 2149-2154.
18. Pfaff E.
   EMBO J., 1982, 1, 869-874.
19. Vaimus et al.
   Nature, Vol 333, p. 504.

## Revendications

1. Composition immunogène caractérisée en ce qu'elle contient un peptide, ou une séquence peptidique apte à induire une réponse immunitaire conduisant à la production d'anticorps Capables d'inhiber l'infection par un HIV, ledit peptide présentant un pourcentage d'au moins 60 % d'isologie ou, selon le cas, de 70 % d'homologie avec une séquence peptidique, protéique ou glycoprotéique issue de HIV.

2. Composition selon la revendication 1 caractérisée en ce que la réponse immunitaire est une réponse in vivo capable d'inhiber l'infection par HIV.

3. Composition selon l'une des revendications 1 ou 2 caractérisée en ce que le peptide utilisé présente un pourcentage d'au moins 60 % d'isologie ou, selon le cas, de 70 % d'homologie avec une séquence d'acides aminés d'un gp 160 env ou p 27 Nef de HIV.

4. Composition selon l'une des revendications 1 à 3 caractérisée en ce que le peptide contient de 5 à 25 acides aminés, notamment de 8 à 15 acides aminés, possédant l'une des séquences suivantes d'acides aminés :
G V V S T P L V N L I N G Q
G V V S T P L V N L I N
K M D G K L T G V V S T P L V N L I N G Q
G I V R T P L A E L L D G E
ou une séquence soit plus longue, soit plus courte mais comprenant le site qui confère au peptide correspondant la capacité d'introduire in vivo la production d'anticorps ayant la propriété d'inhiber une infection de lymphocytes T par HIV.

5. Peptide caractérisé en ce qu'il comprend la séquence suivante d'acides aminés :
S K S S V T G W P

6. Composition immunogène comprenant le peptide :
S K S S V T G W P
ledit peptide présentant au moins 60 % d'isologie, ou 70 % d'homologie avec un peptide issu d'une protéine nef de HIV.
